# EUROPEAN PATENT APPLICATION

(11) **EP 4 393 594 A2**
(43) Date of publication of application: **03.07.2024**
(21) Application number: 23209365.8
(22) Date of filing: 13.11.2023
(51) Int. Cl.: B01L 3/00, A61B 10/00, G01N 33/558

(54) **TEST DEVICE**

(30) Priority: 09.12.2022 CN 202211585604; 28.12.2022 US 202263435655 P; 04.01.2023 GB 202300079
(71) Applicant: Hangzhou Biotest Biotech Co., Ltd., Yuhang District Hangzhou (CN)
(72) Inventor: WU, Shujiang, Hangzhou (CN); ZHAO, Qihui, Hangzhou (CN); GENG, Hui, Hangzhou (CN); HONG, Liang, Hangzhou (CN)
(74) Representative: Piotrowicz, Pawel Jan Andrzej

(57) **Abstract**

The present invention provides a device for testing an analyte in a liquid sample, including: a detection chamber, the detection chamber being used for receiving a testing element, and the testing element being configured to test an analyte in the liquid sample; and a collector, an absorption element on the collector absorbing the liquid sample, and the collector being accommodated by the test device. The device can realize liquid self-testing, such as collection and self-testing of a saliva sample.

## Description

### CROSS-REFERENCE TO RELATED APPLICATIONS

The present application claims the priorities of Chinese patent application No. 2022115856048 filed on December 9, 2022, the US provisional application No. US63/435,655 filed on December 28, 2022, the UK patent application No. GB2300079.7 filed on January 4, 2023, the entire contents of which, including but not limited to abstracts, accompanying drawings, specifications and claims, are a part of the present invention.

### BACKGROUND OF THE INVENTION

### Field of the Invention

The present invention relates to a device for collecting a liquid sample, in particular to a device for collecting and testing an analyte in a liquid sample in the field of rapid diagnosis, such as a urine and saliva collection and test device.

### Description of the Related Art

The following description is merely an introduction of some background knowledge and does not constitute any limitation to the present invention.

At present, the test device for detecting the presence or absence of an analyte in sample is widely used in hospitals or homes, and such test devices for rapid diagnosis include one or more test strips, for example, for early pregnancy testing and drug of abuse testing. Such test devices for rapid diagnosis is very convenient, and can obtain a test result from the test strips in one minute or at most ten minutes or so.

Drug tests are widely used by the drug control department, the Public Security Bureau, drug rehabilitation centers, physical examination centers, physical examination offices of national conscription, etc. Drug tests are diverse and frequent. In some cases, samples need to be collected and then tested by professional testing agencies or test laboratories. In some cases, tests need to be completed on the spot in time. For example, persons who drive after drug use (referred to as "Drugged Driving") need to be tested on the spot, to obtain the test results in time.

For example, testing of saliva samples is gradually accepted and favored by testing agencies or testing personnel due to convenient collection. In some literatures, various specimen collection and test devices for clinical and domestic uses have been described. For example, the US Patent No. 5,376,337 discloses a saliva sampling device in which a piece of filter paper is used for collecting saliva from the mouth of a subject and delivering the saliva to an indicator reagent. US patents US 5,576,009 and US 5,352,410 each disclose a syringe-type liquid sampling device.

In addition, at-home self-testing is increasingly common, and many self-test products appeared for COVID-19. As at-home testing of infectious diseases is increasingly popular, it is necessary to design the existing products such that they can realize at-home self-testing, such as at-home self-testing of drug of abuse or testing with a small amount of sample.

In view of the above technical problems in some conventional products, it is necessary to improve them to provide an alternative approach to solve the defects of the prior art, especially a drug self-test device or method.

### BRIEF SUMMARY OF THE INVENTION

In order to overcome the defects of the prior art, the present invention provides a device for testing an analyte in a sample, which can realize self-sampling and self-testing and requires a small amount of sample. The sample here may be a liquid sample, or a solid sample such as a solid powder sample.

In a first aspect, the present invention provides a device for testing an analyte in a fluid sample, including a detection chamber. The detection chamber is used for receiving a testing element, and the testing element is configured to test the analyte in the liquid sample.

In some embodiments, the device further includes a piercing element. The piercing element is in fluid communication with the testing element.

In some embodiments, the piercing element is configured to pierce a chamber containing a treatment liquid. In some embodiments, the piercing element and the testing element are in fluid communication through a water absorbent diversion material. In this way, after the chamber is pierced, the treatment liquid flows onto the water absorbent material and then onto the testing element under the capillary action.

In some embodiments, the piercing element is located upstream of the testing element, or the water absorbent diversion material is located upstream of the piercing element. In some embodiments, one end of the water absorbent material is connected or in fluid communication with a sample application area of the testing element. In some embodiments, the water absorbent diversion material is provided with a hole which allows the piercing element to run through.

In some embodiments, the piercing element is capable of piercing the chamber and guiding the fluid to flow onto the water absorbent diversion material, so that when the chamber is pierced by the piercing element, the treatment liquid flows onto the water absorbent material along the diversion structure.

In some embodiments, the treatment liquid is sealed in a chamber. In some embodiments, the test device includes a collector for collecting a sample, and the chamber containing the treatment liquid is located on the collector. The sample here may be a liquid sample, or any solid sample such as a powder sample.

In some embodiments, the chamber containing the treatment liquid is arranged on the test device. The chamber has a first state that seals the treatment liquid, and a second state that is not sealed. In some embodiments, the chamber containing the treatment liquid is in fluid communication with the testing element or located upstream of the testing element, and when the chamber containing the treatment liquid is opened to be in an unsealed state, the treatment liquid in the chamber flows onto the testing element. In some embodiments, the opening the chamber to make it unsealed is completed by the collector. In some embodiments, the chamber containing the treatment liquid includes a piston. The treatment liquid is sealed in the chamber by the piston. At this time, the chamber is in the first state. When the piston moves from the first position that is sealed to the second position, the sealed chamber is opened, so that the treatment liquid flows out of the chamber and then onto the testing element. In some embodiments, the piston can be pushed by a push rod on the collector. In some embodiments, the collector includes an absorption element for absorbing the liquid sample and a push rod for pushing the piston to move. While the collector collects the sample and puts it in the test device, the absorption element contacts the sample application area of the testing element, and then pushes the piston to move together with the push rod. In some embodiments, when the collector returns to the same accommodating area on the test device, the whole collector is pushed to move. This movement causes the push rod to push the piston to move, such that the treatment liquid is released. This movement also causes the absorption element to be squeezed and release the liquid sample onto the testing element. In some embodiments, the piston includes piston sealing ends and a piston rod connecting the sealing ends. The treatment liquid is provided between the two sealing ends. The treatment liquid is sealed in the treatment liquid chamber by the piston. In some embodiments, an end of the chamber has an opening. When the piston is in the first position, the piston is away from the opening; and when the piston is in the second position, the opening is located between the two sealing ends. In some embodiments, the sealing ends are elastic seals, and the elastic seals are fitted with an inner surface of the chamber to form a sealed space that contains the treatment liquid therein.

In some embodiments, the sample application area is located downstream of the chamber containing the piston. In some embodiments, the chamber containing the piston is in fluid communication with the testing element through the water absorbent diversion element.

In some embodiments, the device includes a bottom plate and an upper cover, and the testing element and the water absorbent material is located between the bottom plate and the upper cover. In some embodiments, the bottom plate is provided with an area for accommodating the testing element and an area for accommodating the water absorbent diversion material.

In some embodiments, the piercing element is arranged on the bottom plate. In some embodiments, the upper cover includes a piercing area, and the piercing element is located in the piercing area. The upper cover further includes a test result reading area, and a testing area of the testing element is located in the test result reading area. In some embodiments, the chamber containing the piston is located between the upper cover and the bottom plate.

In some embodiments, the device further includes a first accommodating area for receiving the sample collector. When the sample collector is received, accommodated or contained by the test device, the collector is located on the test device. After leaving the first accommodating area on the test device, the collector collects the sample and then returns to the second accommodating area on the test device to start testing. Starting testing is to allow the sample on the collector to contact the testing element so as to start testing or assaying. In some embodiments, the first accommodating area and the second accommodating area may be areas in different positions or in the same position. That is, the first accommodating area and the second accommodating area are in the same position on the test device, or optionally, the second accommodating area and the first accommodating area are in different positions on the test device. The "different" here may be partially different and partially the same.

In some embodiments, the first or second area for accommodating the sample collector on the test device includes an absorption element accommodating area for accommodating the absorption element on the collector, and/or a handle area for accommodating a handle of the collector. In some embodiments, the collector has a state in which no sample has been collected (at this time, the collector is in the first state of the accommodating area of the test device), and a state in which the collector leaves the test device to collect the sample, and a state in which testing is started after the collector returns to the test device to be accommodated by the test device or while it is accommodated by the test device (the second state of the collector). In some embodiments, the first state and the second state of the collector both occur in the same accommodating area on the test device, but at different times. In some embodiments, the first state and the second state of the collector both occur in the same accommodating area on the test device. For example, before collecting the sample, the collector is accommodated by the accommodating area of the test device; and after leaving the test device and collecting the sample, the collector returns to the same accommodating area to start testing. In some embodiments, the first state and the second state of the collector occur in different accommodating areas on the test device. For example, before collecting the sample, the collector is accommodated by the first accommodating area of the test device; and after leaving the test device and collecting the sample, the collector returns to the second accommodating area to start testing. The first accommodating area and the second accommodating area are in different positions on the test device.

In some embodiments, the handle of the collector includes the chamber containing the treatment liquid. In some specific embodiments, the device includes first and second areas for accommodating the absorption element of the collector. When the absorption element of the collector is located in the first accommodating area, the handle of the collector covers the test result reading area. In this case, the test result reading area is used for accommodating the handle area of the collector. When the absorption element of the collector is located in the second accommodating area, the handle of the collector is accommodated by the piercing area. In this case, the piercing area is used for accommodating the handle of the collector. In this case, if the handle includes the sealed chamber containing the treatment liquid, then the piercing element pierces the chamber containing the treatment liquid such that the treatment liquid is released. In some embodiments, when the absorption element of the collector is located in the first accommodating area, the collector is in the first state prior to absorbing the liquid sample, and when the absorption element of the collector is located in the second accommodating area, the collector is in the second state in which the sample is collected and testing is started. In some embodiments, the first and second areas for accommodating the absorption element of the collector are contained between the test result reading area and the area containing the piercing element. In some embodiments, the first area for accommodating the absorption element of the collector is adjacent or close to the piercing area, and the second area for accommodating the absorption element of the collector is close or adjacent to the test device reading area. The area for accommodating the collector here is realized by different accommodating areas on the test device.

Of course, in some embodiments, the same accommodating area is used to accommodate the collector, no matter what state the collector is in. For example, when the collector is in the first state, in which the collector is located on the test device, and has not absorbed the liquid sample but is ready to collect the liquid sample, the collector is located in the first accommodating area on the test device. This area generally includes the area for accommodating the absorption element of the collector and the area for accommodating the handle. After the collector leaves the test device and collects the fluid sample, it returns to the first accommodating area and starts testing. The first accommodating area here includes the area for accommodating the absorption element of the collector and the area for accommodating the handle of the collector. In this case, in both the first state and the second state, the collector is accommodated by the same accommodating area, i.e., the same position, but the collector has different states. When the collector is in the first state, the absorption element on the collector is dry and does not contain a liquid sample, or the absorption element does not carry a sample, such as a solid or powder sample. When the collector is in the second state, the absorption element of the collector has absorbed a liquid sample, or the absorption element carries a sample, such as a powder sample or a solid sample adsorbed thereon.

In some embodiments, on the device, two areas for accommodating the absorption element of the collector are arranged between the test result reading area and the piercing area. These areas are used for accommodating the absorption element of the collector, so that when the absorption element is accommodated, the absorption element can be compressed so as to release a liquid sample, such as a saliva, urine or blood sample. In some embodiments, the area for accommodating the absorption element includes some snap-fit structures which can compress the absorption element, such an area for the accommodating the absorption element serves as the accommodating area for starting testing.

In some embodiments, the collector includes a chamber containing the treatment liquid, which can be pierced by the piercing element. In some embodiments, when the collector is received by the second or second accommodating area of the test device, the chamber containing the treatment liquid on the collector is pierced by the piercing element so as to release the treatment liquid. At this time, the absorption element of the collector is squeezed or compressed downstream of the piercing element to release the liquid sample. Thereby, the treatment liquid released flows from the upstream water absorbent diversion material to the downstream absorption element so as to contact the absorption element or be mixed with the released liquid sample, thereby flowing to the testing area downstream of the sample receiving area of the testing element. Of course, after the absorption element on the collector contacts the sample application area of the testing element, the treatment liquid will flow to the application area, so that the sample on the absorption element can be dissolved and eluted, and the eluted sample can be driven to flow on the testing element, thereby completing the complete testing of the analyte.

In some embodiments, when the absorption element is accommodated by the accommodating area on the test device, the absorption element contacts the sample application area of the testing element, so that the liquid sample released by compression flows onto the sample application area to start testing.

In some embodiments, the further includes a covering element. When the collector is located in the first accommodating area of the device, the covering element is located in the second accommodating area of the device. When the collector is located in the second accommodating area, the covering element leaves the second accommodating area. In this way, before the collector collects the sample, the covering element can cover some areas where the internal structure is not expected to be seen. For example, if the second accommodating area has a piercing element, then the covering element covers the areas containing the piercing element, which can protect these areas and also prevent the piercing structure in these areas from injuring the working personnel. This can also avoid injuring the operator in a case of at-home self-testing. If the collector contains the chamber containing the treatment liquid, when the collector is located in the second accommodating area, testing is started. At this time, the test result reading window is covered by the covering element. After the result is read, the covering element is allowed to cover the test result reading area, and the whole test device is discarded.

In some embodiments, the covering element leaving the second accommodating area may cover the first accommodating area at an appropriate time. That is, after the testing is completed and the test result is read, the covering element is located on the first accommodating area, or covers the test result reading area.

In a second aspect, the present invention provides a collector. The collector includes an absorption element. The absorption element is used for collecting a sample. For example, the absorption element has an absorption side for collecting a liquid sample. Of course, the absorption element is also capable of adsorbing a solid, such as a powder sample. In some embodiments, the collector further includes a treatment liquid, which functions to be mixed with the sample to treat the sample. In some embodiments, the treatment liquid is accommodated by a chamber which is located on the collector. In some embodiments, one end of the collector includes the absorption element, and the other end includes the chamber containing the treatment liquid. In some embodiments, the absorption element on the collector is fixed at one end of the collector, and the chamber containing the treatment liquid on the other end and the absorption element are located in the same direction or the same plane of the collector.

In some embodiments, the absorption element of the collector is fixed in the chamber at the end of the collector, and a part of the absorption element is exposed to absorb the liquid sample. In some embodiments, the absorption element is fixed by a fixing element, and the fixing element is inserted into the chamber such that the absorption element is fixed in the chamber. In some embodiments, the absorption element has three independent absorption heads to absorb the liquid sample or stick the solid powder. In some embodiments, the absorption element can be compressed. In some embodiments, the fixing element is elastic, and the absorption element is fixed into the chamber through an elastic fixing structure. In some embodiments, the fixing element is an elastic clip. After the clip holds the absorption element, the absorption element is inserted into the fixed chamber so as to be clamped in the fixed chamber. In some embodiments, the absorption element is bonded to one end of the collector, so that the absorption element can absorb a liquid sample or stick a solid sample, such as a powder sample. In some embodiments, one end of the collector has a protruding plane to which the absorption element is stuck. In some embodiments, the absorption element includes sponge, filter paper and other materials that can absorb a liquid sample or stick a solid sample. The "absorption" here includes absorption of a sample by capillary action, as well as in the general sense of allowing a liquid to enter the absorption element, and allowing a solid or a solid powder to be stuck the absorption element. Unlike absorption in the usual sense, the absorption in the present invention includes adsorption as well.

In some embodiments, the collector may be used to take a trace amount of liquid sample, such as saliva or slobber, from the oral cavity. In some embodiments, the collector has two faces. The absorption element and the sealed chamber containing the treatment liquid are located on the same face. In this case, when the collector is accommodated by the second area of the device, the sealed chamber containing the treatment liquid just faces the piercing element, and the absorption element is clamped on the clamping structure, so that the absorption element is compressed to release the liquid sample to the sample application area of the testing element.

In a third aspect, the present invention provides a method for testing an analyte in a fluid sample, including:

providing a device, including: a detection chamber, the detection chamber being used for receiving a testing element, and the testing element being configured to test the analyte in the liquid sample; and a sample collector, including a sample absorption element and a sealed chamber containing a treatment liquid.

The absorption element of the collector is allowed to absorb the liquid sample and be accommodated by an accommodating area of a detection chamber, so that the absorption element is compressed to release the liquid sample. Moreover, the sealed chamber is pierced by a piercing element arranged in the detection chamber so as to release the treatment liquid.

In some embodiments, the absorption element is accommodated by the accommodating area on the test device, and the chamber containing the piercing element is allowed to accommodate the sealed chamber containing the treatment liquid on the collector. Thereby, when a pressure is applied to the collector, the sealed chamber is pierced by the piercing element to release the treatment liquid, and the absorption element is compressed in the accommodating area. In some embodiments, the chamber accommodating the absorption element includes a sample application area of the testing element, so that the absorption element can contact the sample application area of the testing element and be compressed in this area.

The sealed chamber of the collector is allowed to be located upstream of the area accommodating the absorption element, or the piercing element is allowed to be located upstream of the sample application area of the testing element. The piercing element is arranged on or in the second accommodating area. In some embodiments, the treatment liquid is allowed to flow from upstream to downstream and be mixed with the liquid sample. In some embodiments, a diversion element is arranged upstream of the testing element of the detection chamber, so that the treatment liquid can flow to the sample application area of the testing element through the diversion element. In some embodiments, the absorption element of the collector is allowed to go deep into the oral cavity to take a saliva sample in the oral cavity.

In some embodiments, the detection chamber is provided with an area for accommodating the absorption element of the collector, and a first area for accommodating the sealed chamber containing the treatment liquid on the collector. When the collector is located in the first area, the absorption element of the collector does not contain a sample. The area for accommodating a handle of the collector includes a test result area, which is located in a result reading area. The test result reading area accommodates the sealed chamber containing the treatment liquid on the collector. The collector is allowed to leave the test device, and the absorption element is allowed to absorb or adsorb a sample. At this time, the result reading area is exposed. Then, the collector is allowed to return to the second accommodating area on the test device. The second accommodating area includes an accommodating area for accommodating the absorption element and an area for accommodating the sealed chamber containing the treatment liquid. The second accommodating area contains a piercing element for piercing the sealed chamber containing the treatment liquid.

In some embodiments, the chamber containing the piercing element and the test result reading chamber are located at the ends of the test device, and the chamber accommodating the absorption element is located between the chamber containing the piercing element and the test result reading chamber.

In a fourth aspect, the present invention provides a method for testing an analyte in a fluid sample, including:

providing a device, including: a detection chamber, the detection chamber being used for receiving a testing element, and the testing element being configured to test the analyte in the liquid sample; and a sample collector, the collector including an element for absorbing or adsorbing the sample. The test device includes an accommodating chamber for accommodating the absorption element on the collector.

The absorption element of the collector is allowed to absorb the liquid sample and be accommodated by the accommodating area on a detection chamber, so that the absorption element is compressed to release the liquid sample and contact the sample application area of the testing element located in the accommodating chamber.

In some embodiments, the test device further includes a chamber containing a treatment liquid, which has a movable piston therein. In an initial position, the piston seals the treatment liquid in the chamber. After the collector is allowed to return to the test device and the absorption element is allowed to be accommodated by the accommodating chamber again, the collector is pushed to move, so that the collector pushes the piston in the chamber containing the treatment liquid to move, and thereby, the treatment liquid is released into the test device. The collector is pushed, so that the absorption element is compressed to release the sample onto the sample application area of the testing element. Alternatively, the collector is pushed, so that the treatment liquid is released from the chamber, and then flows to the absorption element and dissolves the sample on the absorption element.

In all the above embodiments, the sample is a blood sample or a saliva sample. In some embodiments, the analyte is a virus, a bacterium, a fungus, or a small molecule compound such as THC.

### Beneficial effect

The above structure can be used for testing an analyte in a liquid sample, especially for taking samples from an oral cavity. It is only required to scrape or slightly scrape the oral cavity with the collector, and then the absorption element can absorb the saliva in the oral cavity. The structure has the advantages of simple sampling and convenient operation, and can realize self-testing (OCT) or POCT (point-of-care testing) through a professional agency.

### BRIEF DESCRIPTION OF THE DRAWINGS

FIG. 1 is an assembled view of a test device in a specific embodiment of the present invention (an assembled view as delivered, in a non-testing state), where a covering element is in an accommodating area containing a piercing element, and part of a collector covers a test result reading area;
FIG. 2 is a schematic exploded structural view of a test device in a specific embodiment of the present invention (in a testing-started state);
FIG. 3 is a schematic exploded structural view of an upper cover, a bottom plate and a testing element of a test device in a specific embodiment of the present invention;
FIG. 4 is a schematic three-dimensional assembled view of a bottom plate containing a testing element, a diversion element and piercing elements thereon in a specific embodiment of the present invention;
FIG. 5.1 is a schematic three-dimensional assembled view of a testing element and a diversion element in a specific embodiment of the present invention;
FIG. 5.2 is a schematic three-dimensional structural view of an upper cover in a specific embodiment of the present invention;
FIG. 6 is a schematic three-dimensional structural view of a back side of an upper cover (in a direction facing the bottom plate) in a specific embodiment of the present invention;
FIG. 7 is a schematic three-dimensional structural view of a test device assembled from an upper cover, a bottom plate and a testing element in a specific embodiment of the present invention;
FIG. 8 is a schematic three-dimensional structural view of a collector in a specific embodiment of the present invention (with the chamber containing the treatment liquid being unsealed);
FIG. 9 is a schematic three-dimensional structural view of a clamping element clamping absorption elements in a specific embodiment of the present invention;
FIG. 10 is a schematic view showing the complete structure of the collector in a specific embodiment of the present invention (with the chamber containing the treatment liquid being sealed by a sealing film);
FIG. 11 is a schematic structural view of a collector of a test device during testing in a specific embodiment of the present invention, where the collector covers the piercing area and the testing area is exposed such that the test result can be read;
FIG. 12 is a schematic three-dimensional structural view of a test device in a specific embodiment of the present invention, where the collector is located in an accommodating area on the test device (before testing is started);
FIG. 13 is an exploded structural view of a test device in a specific embodiment of the present invention;
FIG. 14 is a schematic three-dimensional structural view of an upper cover of a test device in a specific embodiment of the present invention;
FIG. 15 is a schematic three-dimensional structural view of a collector in a specific embodiment of the present invention;
FIG. 16A is a schematic three-dimensional structural view of a test device in a specific embodiment of the present invention, where the collector is located in an accommodating area on the test device to start testing;
FIG. 16B is a schematic structural cutaway view of a test device in a specific embodiment of the present invention, where the collector is located in an accommodating area on the test device to start testing;
FIG. 17 is a schematic three-dimensional structural view of a test device and a collector assembled in a specific embodiment of the present invention;
FIG. 18 is a schematic exploded structural view of a test device and a collector in a specific embodiment of the present invention;
FIG. 19 is a schematic three-dimensional structural view of a collector in a specific embodiment of the present invention;
FIG. 20 is a schematic view showing positions of a testing element on a bottom plate and a sealed chamber containing a piston in a specific embodiment of the present invention;
FIG. 21 is a schematic three-dimensional structural view showing positions and fits between a testing element on a bottom plate and a sealed chamber after or before the collector starts testing in a specific embodiment of the present invention;
FIG. 22 is a schematic structural cutaway view of a collector before testing is started in a specific embodiment of the present invention; and
FIG. 23 is a schematic structural cutaway view of a test device after the collector starts testing in a specific embodiment of the present invention.

### DETAILED DESCRIPTION OF THE INVENTION

The structures involved in the present invention or the technical terms used are further explained below. Unless otherwise specified, they shall be understood and explained according to the general terms commonly used in the art.

### Testing

Testing means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, testing means testing an amount of a substance or material. Further, assay also means immunoassay, chemical assay, enzyme assay, and the like.

### Samples

Specimens tested or collected by the test device of the present invention include biological liquids (for example, case liquids or clinical specimens), such as liquid samples or solid samples. Liquid specimens or liquid samples, or fluid samples or fluid specimens may be derived from solid or semi-solid specimens, including feces, biological tissues and food specimens. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. Food specimens include food processed materials, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other waste water. The solid samples here include specimens containing no water or little water, such as solid soil, animal and plant tissues, or drug powder, such as drug of abuse powder samples.

An appropriate test device or testing element according to the present invention can be used to detect any analyte. Preferably, the test device of the present invention is used to test drug small molecules in saliva or urine, or viruses in powder specimens or blood samples, such as human immunodeficiency virus (HIV) and coronaviruses. Of course, a collection device or collector 103 according to the present invention can be used to collect samples in any form, regardless of being solid or liquid, as long as these liquids or liquid samples can be absorbed, stuck and adsorbed by absorption elements 1031, 2061, 3031, or these powdery specimens can be stuck or scraped by the absorption elements of the collector. The absorption elements here may be made of water absorbent materials such as sponge, polyester fiber, filter paper, foam, and flocking.

### Treatment liquid

A treatment liquid is a solution or reagent used to treat a liquid sample. Different from the liquid sample, the treatment liquid is generally a solution prepared in advance, and can elute, dissolve or treat the absorption element, or treat the liquid sample, for example, adjust the pH, so as to reduce nonspecific binding, avoid false positives or false negatives, or improve the properties of immunoassay of an analyte in a sample on a test strip. Generally, the treatment liquid does not include the analyte, nor does it include components with the same properties as the sample. Therefore, after the treatment liquid is mixed with the liquid sample, the new mixed solution formed can flow to a detection chamber to contact the testing element. Of course, the treatment liquid here functions only to dissolve a solid sample into a liquid sample so that the sample can flow on the testing element and be tested. The treatment liquid here may only be a liquid, such as water, pure water, deionized water, alcohol and other liquids. When solid powder is stuck to the absorption element, the treatment liquid contacts the absorption element to dissolve the solid powder sample stuck to the absorption element, and then the solid sample is dissolved in the treatment liquid to form a liquid sample.

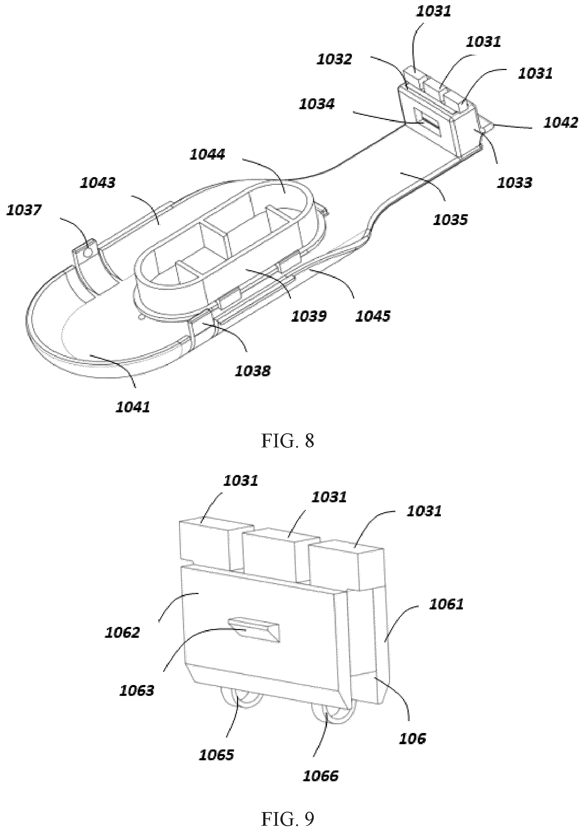

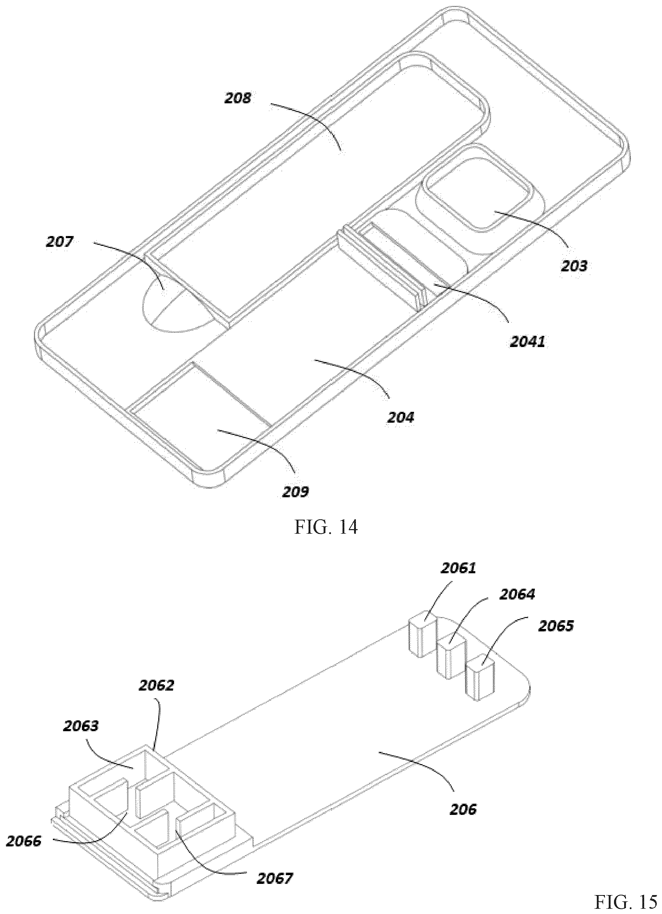

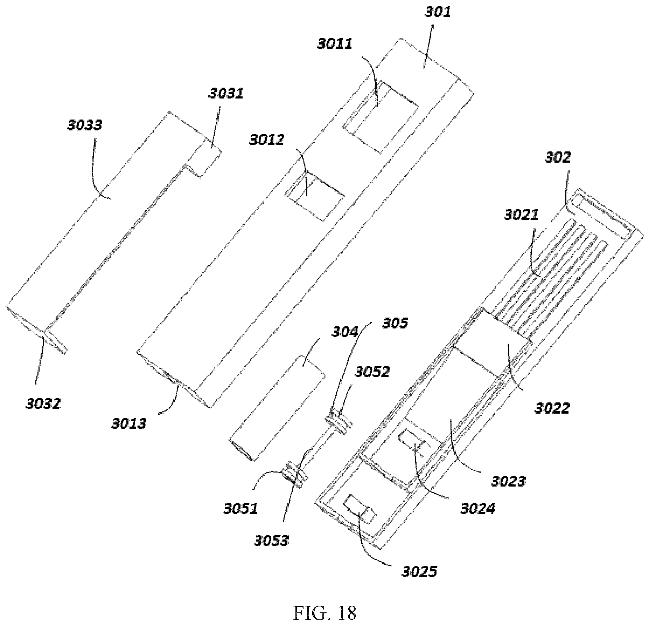

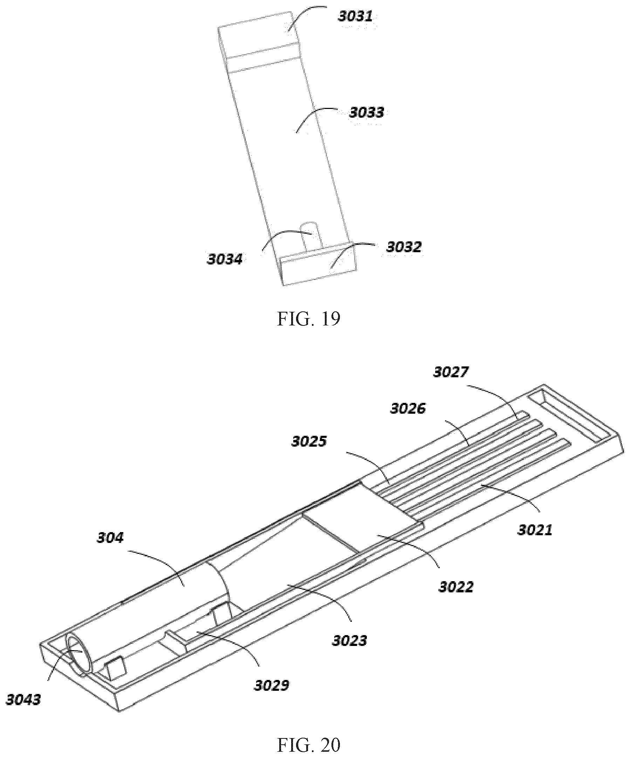

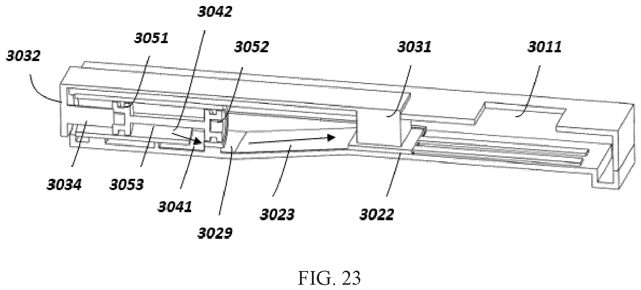

The treatment liquid here may be accommodated in a chamber, for example, a chamber 1039 shown in FIG. 8. This chamber has a buffer chamber 1044 in which the treatment liquid is packaged in advance. This chamber may be sealed with a sealing film 1040 that can be pierced easily, such as an aluminum film. When the film is pierced by a piercing element, the treatment liquid can be released from the sealed chamber 1039. The chamber may also be a chamber 2062 shown in FIG. 15, which is filled with the treatment liquid in advance and then sealed with a sealing film. Of course, the treatment liquid may also be included in a tube 304. The tube has a movable piston 305, and the treatment liquid is contained in the chamber defined by the piston. For example, the chamber 304, as shown in FIG. 18 and FIG. 20 to FIG. 23, contains a piston 305. The piston contains piston gaskets 3051, 3052 at two ends, and a piston rod 3053 is arranged between the gaskets. The treatment liquid is sealed between the two gaskets. At the beginning, the piston seals the treatment liquid in the chamber 304. When the piston moves forward, the piston gasket passes through an opening 3041, so that the treatment liquid flows out of the opening 3041. Specific embodiments will be described in detail below.

### Downstream and upstream

Downstream or upstream is divided according to a flow direction of a liquid. Generally, a liquid flows to a downstream area from an upstream area. The downstream area receives the liquid from the upstream area, and a liquid also may flow to the downstream area along the upstream area. Here, downstream or upstream is generally divided according to a flow direction of a liquid, for example, on some materials where capillary force is utilized to promote the flow of a liquid, a liquid may overcome gravity to flow towards an opposite direction to the gravity; and in this case, downstream or upstream is divided according to a flow direction of the liquid. For example, in the test device of the present invention, after the absorption element 1031 absorbs a fluid sample or a sample, the fluid sample may be compressed out of the absorption element. In some embodiments, the fluid sample compressed out contacts a sample application area 1083 of a testing element. In this case, the fluid sample can flow from the upstream sample application area to the downstream testing area 1082, and finally to a water absorption area 1081. In a circulation process, the fluid sample flows from a label area to a testing area 1082 on which a detection area 1087 and a test result control area 1088 are provided. The testing area may be a polyester fiber film, and the sample application area may be a glass fiber. In some embodiments, upstream of the sample application area 1076, an absorbed water diversion area is further provided, on which a diversion material 1084 is provided. This water absorbent material is fixed onto a bottom plate and connected with piercing elements 1012, 1013. When the piercing element pierces the chamber of the treatment liquid, the treatment liquid released flows to the diversion material 1084, and the treatment liquid flowing to the diversion material flows to the downstream sample application area 1083 along the diversion material, and then is mixed with the sample flowing to the sample application area 1076 to flow to the downstream testing area 1082.

### Gas communication or liquid communication

Gas communication or liquid communication means that a liquid or gas can flow from one place to another. In the flow process, the liquid or gas may pass through some physical structures that play a guiding role. The "pass through some physical structures" here means that the liquid passes through the surface of these physical structures or the internal space of these physical structures and flows to another place passively or actively, where the passive flow is usually caused by external forces, such as the flow under the capillary action. The flow here may also be a flow due to self-action (gravity or pressure) of the liquid or gas, and also may be a passive flow. The communication here does not mean that a liquid or a gas is necessarily present, but indicates a relationship or state between two objects under some circumstances. If a liquid is present, it can flow from one object to another. Here it means the state in which two objects are connected. In contrast, if there is no liquid or gas communication state between two objects, and a liquid exists in or on one object but is unable to flow into or on another object, it is a non-communication, non-liquid communication or non-gas communication state.

### Detachable combination

A detachable combination means that two components are connected in several different states or positional relationships. For example, with two components being physical components, they can be separated at the beginning and then connected or combined in an appropriate first case, and separated in an appropriate second case. Physically, such separation is spatial separation without contact. Alternatively, the two components are combined at the beginning, and can be physically separated from each other when appropriate. Alternatively, two objects are separated at the beginning, combined to achieve a specified function if necessary, then separated, or later combined again for a purpose. In short, combination or separation of two components or two objects can be easily made and repeated many times. Of course, the combination or separation can also be single-use. In addition, such combination can be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves can be detached or can be indirectly combined by other objects.

### Testing element

The "testing element" used herein refers to an element that can be used to detect whether a sample or a specimen contains an interested analyte. Such testing can be based on any technical principles, such as immunology, chemistry, electricity, optics, molecular science, nucleic acids, and physics. The testing element can be a lateral flow test strip that can detect a variety of analytes. Of course, other suitable testing elements can also be used in the present invention.

Various testing elements can be combined for use in the present invention. One form of the testing elements is a test strip. The test strips used for analyzing the analyte (such as drugs or metabolites that show physical conditions) in samples can be of various forms such as immunoassay or chemical analysis. The test strips may adopt a non-competitive or competitive analysis mode. A test strip generally contains a water absorbent material that has a sample application area, a reagent area, and a testing area. Samples are added to the sample application area and flow to the reagent area under the capillary action. If an analyte exists in the reagent area, samples will bind to the reagent. Then, the samples continue to flow to the testing area. Other reagents such as molecules that specifically bind to the analyte are immobilized in the testing area. These reagents react with the analyte (if any) in the sample and bind to the analyte in this area, or bind to a reagent in the reagent area. The label used to display the detection signal exists in the reagent area or the detached label area.

In a typical non-competitive analysis mode, if a sample contains the analyte, a signal will be generated; and if not, no signal will be generated. In a competitive method, if no analyte exists in the sample, a signal will be generated; and if the analyte exists, no signal will be generated.

The testing element may be a test strip, which may be made of a water absorbent material or non-water absorbent material. The test strip may contain several materials used for delivery of liquid samples. One material of the test strip can cover the other material thereof. For example, the filter paper covers the nitrocellulose membrane. One or more materials may be used in one area of the test strip, and one or more other different materials may be used in the other area. The test strip can be stuck to a certain support or on a hard surface for improving the strength of holding the test strip.

The analyte is detected through a signal generating system. For example, one or more enzymes that specifically react with this analyte is or are used, and the above method of fixing a specific binding substance on the test strip is used to fix the combination of one or more signal generating systems in the analyte testing area of the test strip. The substance that generates a signal may be in the sample application area, the reagent area or the testing area, or on the entire test strip, and one or more materials of the test strip may be filled with this substance. The solution containing a signifier is added onto the surface of the test strip, or one or more materials of the test strip is or are immersed in a signifier-containing solution. The test strip containing the signifier solution is dried.

Various areas of the test strip may be arranged in the following way: a sample application area, a reagent area, a testing area, a control area, an area to determine whether the sample is adulterated or not, and a liquid sample absorption area. The control area is located behind the testing area. All areas can be arranged on one test strip that is only made of one material. Alternatively, different areas may be made of different materials. Each area can be in direct contact with the liquid sample, or different areas are arranged according to the flow direction of liquid sample; and a tail end of each area is connected and overlapped with the front end of another area. Materials used can be those with good water absorption such as filter paper, glass fibers or nitrocellulose membranes. The test strip may also be in other forms.

The nitrocellulose membrane test strip is commonly used, that is, the testing area includes a nitrocellulose membrane on which a specific binding molecule is immobilized to display the test result; and other test strips such as cellulose acetate membrane or nylon membrane test strips may also be used. For example, test strips and similar devices with test strips disclosed in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620, and US 6403383. The test strips and similar device with test strips disclosed in the above patents may be applied to the testing element or test device of the present invention for the detection of an analyte, for example, the detection of an analyte in a sample.

Test strips used in the present invention may be commonly referred as lateral flow test strips. The specific structure and detection principle of the test strips are well known to a person skilled in the art in the prior art. A common test strip includes a sample collection area or a sample application area, a label area, a testing area and a water absorption area. The sample collection area includes a sample receiving pad, the label area includes a label pad, and the water absorption area may include a water absorbent pad. The testing area includes necessary chemical substances for detecting the presence or absence of the analyte, such as immunoreagents or enzyme chemical reagents. The nitrocellulose membrane test strip is commonly used, that is, the testing area includes a nitrocellulose membrane, and a specific binding molecule is immobilized on the nitrocellulose membrane to display the test result; and other test strips such as cellulose acetate membrane or nylon membrane test strips may also be used. Of course, in the downstream of the testing area, there may also be a test result control area. Generally, the control area and the testing area in the form of horizontal lines, namely, a test line or a control line. Such test strips are conventional. Of course, they may also be other types of test strips for detection under the capillary action. In addition, there are dry chemical reagent components on common test strips, for example, an immobilized antibody or other reagents. When the test strip contacts a liquid, the liquid flows along the test strip under the capillary action, and the dry reagent components are dissolved in the liquid and treated in a next area, and the dry reagents react in the area for necessary detection. The liquid flow mainly relies on the capillary action. Here, all of the test strips can be applied to the test device of the present invention or can be disposed in contact with the liquid samples in a detection chamber or used to detect the presence or absence of an analyte in the liquid samples that enter a detection chamber, or the quantity thereof. Generally, the testing element is configured in the detection chamber. When a fluid sample exists in the detection chamber, it contacts the testing element for assaying or testing. The detection chamber herein includes a bottom plate 101 and a cover plate 102 between which a testing element is provided. In some embodiments, the bottom plate 101 is provided with a groove structure for placing a testing element. One or more testing elements may be arranged to test different types of analytes in one sample, except for the foregoing test strip or lateral flow test strip that contacts the liquid sample itself to test whether the liquid samples contain analytes.

### Analyte

Examples that can use an analyte related to the present invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of abuse of drug include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium^{®}, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the present invention may also be used for detecting drugs belonging to a medical use but easy to be taken excessively, such as tricyclic antidepressants (imipramine or analogues) and acetaminophen. These drugs are metabolized into micromolecular substances after absorbed by human body. These micromolecular substances exist in blood, urine, saliva, sweat and other body fluids or in some body fluids.

For example, the analyte detected by the present invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis*, *Camyplobacter genus, L. monocytogenes, Vibrio,* or *Bacillus cereus)* and substances related with physiological features in a urine sample, such as pH and specific gravity. Chemical analysis of any other clinical urine may be performed by lateral flow detection in combination with the device of the present invention.

### Flow of liquid

Typically, the flow of liquid refers to flow from one place to another. Under normal circumstances, the flow of liquid in nature mostly is from a high place to a low place under the action of gravity. Flow herein also depends on external force (namely, external gravity), and thus it can become flow under normal gravity. In addition to gravity, the flow of liquid may also overcome gravity to move from a low place to a high place. For example, the liquid can flow from a low place to a high place through extraction, compression or pressure received by it. Alternatively, the liquid may flow against its own gravity in relation to pressure.

### Test device

The test device refers to a device for detecting the presence or absence of an analyte in a sample. The test device herein may simply include a detection chamber and a testing element therein, so it can be called a test device. For example, the test device 100 includes a detection chamber which contains a testing element 108. In some embodiments, the detection chamber includes a bottom plate 101 and a cover plate or upper plate 102. The testing element is located in a chamber formed by the bottom plate and the cover plate, or located between the bottom plate and the cover plate. In some embodiments, the bottom plate 101 is provided with a structure for placing the testing element, such as a groove structure 1014 for placing a test strip 108, so that the test strip 108 can be placed on the groove structure. In some embodiments, the bottom plate is further provided with sockets, and the cover plate 102 is provided with corresponding pins. The sockets are fitted with the pins such that the bottom plate and the cover plate are combined together. In some embodiments, a diversion element 1084 is further provided upstream of the test strip 108. The diversion element is an element used for allowing a treatment liquid to flow on to the testing element 108, which is made of a water absorbent material such as glass fiber. In some embodiments, the diversion element 1084 is also arranged on the bottom plate. In some embodiments, on the bottom plate, a piercing area 15 is also arranged in the area upstream of the testing element. The piercing area is provided with piercing elements 1011, 1013, which extend upward from the bottom plate. The diversion element is provided with holes 1086, 1085. In this way, during assembly, the holes 1086, 1085 allow the piercing elements to run through, so that the piercing elements run through the holes 1086, 1085, and protrude outward from the bottom plate. In order to fix the diversion element, the diversion element 1084 may also be provided with a fixing hole 1089 through which a fixing column 1018 runs, so that the diversion element is stably fixed on the bottom plate.

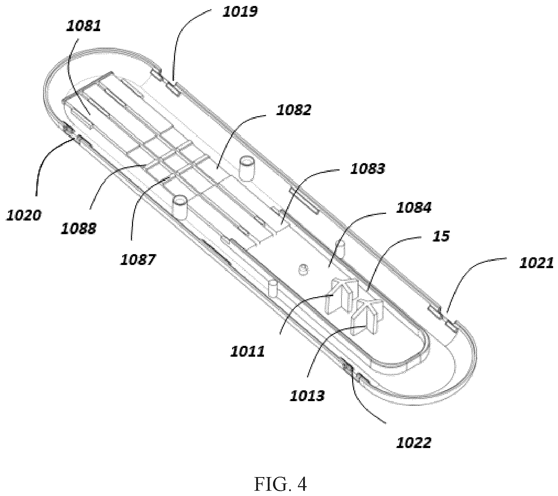

After the piercing elements pierce the chamber containing the treatment liquid, the treatment liquid flows onto the diversion element 1084 along the piercing elements, and then flows onto the sample application area 1083 of the test strip 108 along the diversion element 1084. In order to control the flow speed of the liquid, some pressing structures are arranged on the cover. These structures may apply a pressure to the diversion element, so that the treatment liquid flowing to the diversion element can flow at a lower speed, which prevents excessive liquid from flowing onto the testing element 108, causing flooding and affecting the test result. The testing element 108 is placed downstream of the diversion element 1084 on the bottom plate. The bottom plate 101 is provided with an area 1016 for placing the sample application area 1083 of the testing element 108, an area 1017 for placing a label area on the testing element, an area 1015 for placing a testing area 1082 of the testing element, and an area 1014 for placing a water absorption area 1081 on the testing element. From upstream to downstream of the sample application area of the testing element 108, a sample application pad, a label pad, a nitrocellulose membrane and filter paper may be sequentially overlapped to form the testing element 108. One end of the diversion element 1084 is overlapped by the sample application area 1083 (as shown in FIG. 4), and the nitrocellulose membrane is provided with the testing area.

In some embodiments, the upper plate or cover plate 102 is provided with some structures for fixing the test strip, such as a pressing pin 1029 to apply a pressure to the water absorption area on the testing element, and a pressing block 1030 and an additional parallel pressing block 1031 to apply pressures the overlap between the label pad and the testing pad and the overlap between the label pad and the sample application area. In some embodiments, the cover plate is provided with a blocking structure 1032. When the cover plate 106 is combined and assembled with the bottom plate 105, the blocking structure 1032 is arranged at the junction between the diversion element 1084 and the sample application area 1083 of the testing element, and pressed against the diversion element, thereby preventing the treatment liquid on the diversion element from flowing too fast. Of course, the diversion element can be intentionally folded downstream of the piercing element, which can also reduce the flow speed. This can lengthen the flow path of the treatment liquid on the diversion element, thereby preventing the treatment liquid from reaching the sample application area in advance. In summary, it is necessary to optimize the timing of mixing the fluid sample and the treatment liquid before the diversion element and the absorption element of the collector are compressed, so that the time when the treatment liquid flows to the sample application area 1083 of the testing element through the diversion element 1084 after the piercing element 1011 pierces the sealed chamber 1039 containing the treatment liquid is just consistent with the time when the absorption element 1031 of the collector is compressed to release the liquid to the sample application area 1083. In this way, the treatment liquid and the fluid sample are mixed, and flow to the downstream testing area 1075 at the same time. Of course, this is merely a preferred solution, and it is also possible that the liquid of the absorption element 1031 is compressed and released onto the sample application area 1083 before the upstream treatment liquid flows along the diversion element 1084 to the position where the absorption element 1031 contacts the sample application area 1083. In a preferred embodiment, the absorption element of the collector contacts the sample application area 1083 of the testing element. Of course, the absorption element 1031 may also contact any position on any fluid path from the position where the treatment liquid flows out to the label pad of the test strip, for example, the absorption element may contact the diversion element 1084 or the label pad. In summary, when testing is started, the absorption element of the collector may contact any area downstream of the treatment liquid and be located upstream of the testing area.

Of course, it can be understood that if the absorption element of the collector collects only a small amount of fluid sample, even if the absorption element is compressed, the amount of the liquid flowing to the sample application area of the testing element will be too small to flow to the testing element. If the sample is saliva, which is viscous and not able to flow easily, a treatment liquid is needed to be mixed with the saliva sample to improve the fluidity or increase the total volume of the liquid, so that the liquid is able to flow on the testing element, thereby preventing the amount of liquid from being too small to complete the liquid flow path on the whole testing element. In this case, it is not necessarily particularly stringent, and it is only required that after or while the absorption element contacts the sample application area, the treatment liquid can flow to the sample application area 1083 to contact the absorption element and elute the liquid sample on the absorption element, so that the liquid sample can flow to the downstream testing area for assaying, or the treatment liquid can be mixed with the saliva staying on the sample application area and drive the saliva to flow downstream.

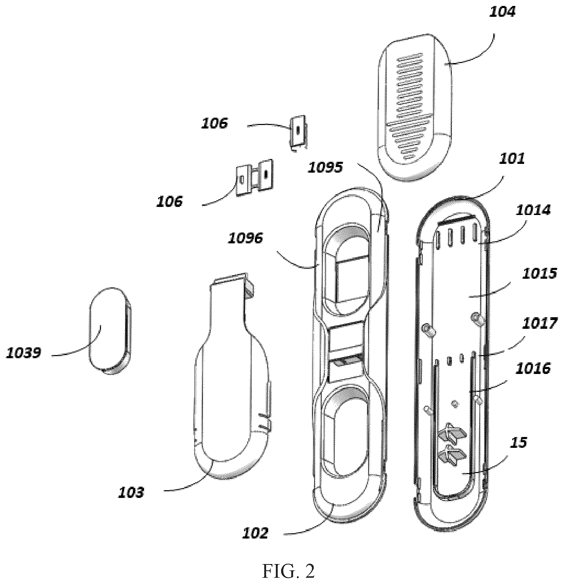

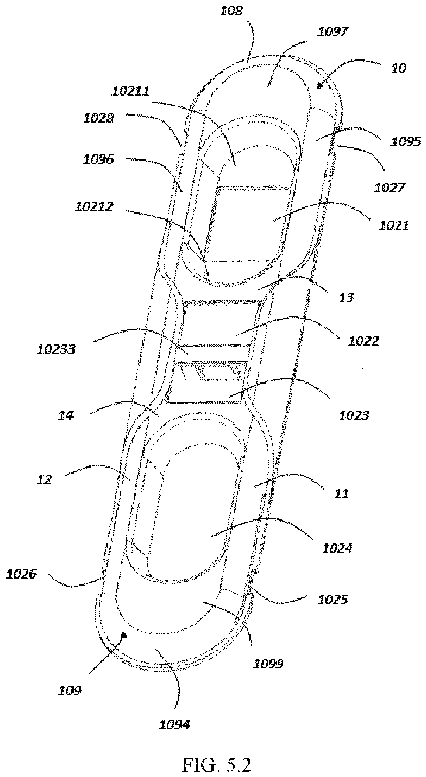

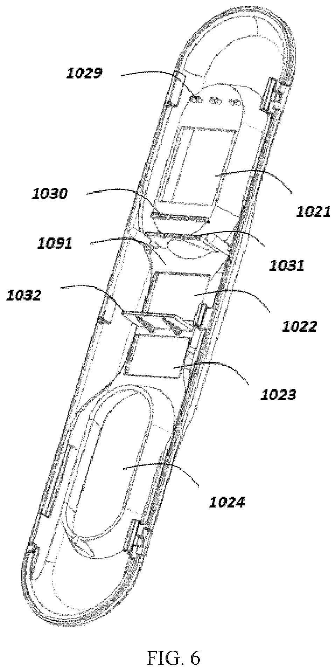

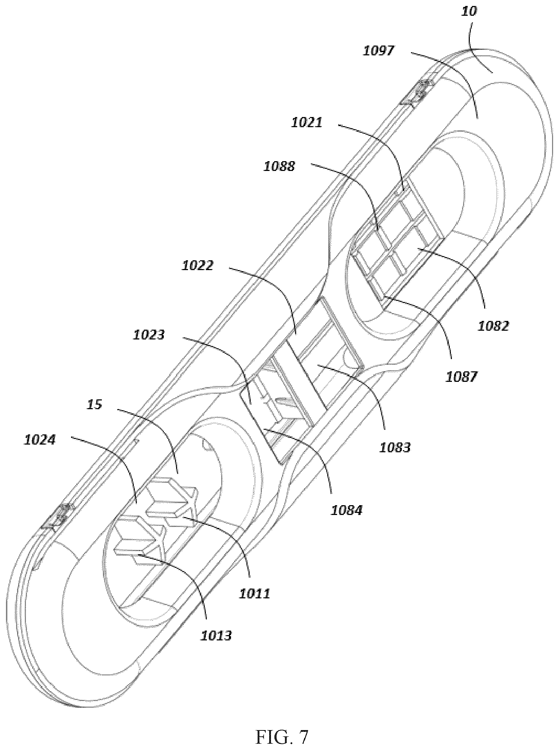

In some embodiments, the test device is further provided with some areas for accommodating the collector. Such areas can allow the collector to be detachably combined with the test device. In case of necessary, the collector is allowed to leave the test device, and in other cases, the collector is allowed to return to the test device. For example, with these areas for accommodating the collector, when the collector is in the initial state, the collector is retained on the test device. When testing is needed, the collector is allowed to leave the test device to collect the sample, and then return to the accommodating area to start testing. After the testing is completed, the collector still stays on the test device. In some embodiments, the upper cover 102 is further provided with an area for accommodating the collector, which is used for accommodating the collector. In some embodiments, one of the areas is an open area 1021 where a test result can be read, as shown in FIG. 2 to FIG 3 and FIG 5.2 to FIG 7, and another area is an area 1024 including the piercing area 15. These two areas may serve as areas for accommodating part of the collector, so that the collector can be detachably arranged in these two areas on the test device.

In some specific embodiments, the two areas 1021, 1024 may exist in the form of chambers. When the upper cover and the bottom plate are combined together, the two chambers each have an opening, and the bottom of the chamber is the bottom plate 101 which serves as the bottom surface of the chamber. Therefore, in some embodiments, the area 1021 contains the testing area of the testing element, and the area 1024 contains the piercing element 1011. From the foregoing description, the testing area is located on the testing element of the bottom plate, and the piercing element extends or protrudes upward from the bottom plate and is located in the area 1024. The upper cover is provided with an opening in the test result reading area 1021. Through the opening, the testing area 1082 of the testing element on the bottom plate 101 can be seen, and at least the test result area 1088 and the control area 1087 on the testing area 1082 can be seen, so that the test result in the testing area can be read. In the present invention, the test result reading area 1021 here has two functions, one of which is to read the test result in the testing area on the testing element, and the other is to accommodate part of the collector. In some embodiments, when the collector is in the initial first state, the area or chamber accommodating the sealed chamber containing the treatment liquid on the collector, or the sealed chamber 1039 containing the treatment liquid on the collector is located in the test result reading area 1021. In some embodiments, the test result reading area 1021 may be located in an accommodating area 10. The accommodating area 10 includes areas 1096, 1095 distributed on two sides of the area 1021, and an area 18 at the tail end. When the sealed chamber 1039 containing the treatment liquid on the collector is located in the area 1021, the other part of the collector covers the area 10. The area 10 further includes an area 1097 to be covered by a cover, which includes the area 1021 to be covered or shielded by the cover plate 104.

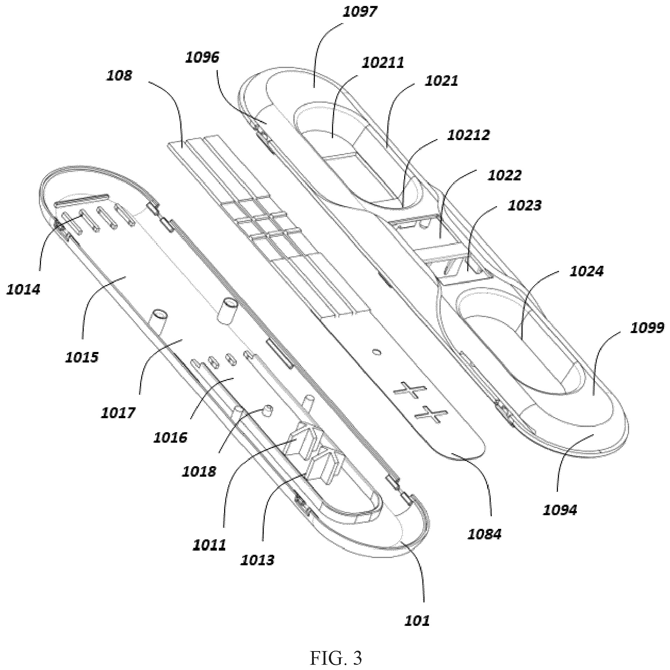

In some embodiments, the collector is typically a flat structure, one end of which is provided with the protruding absorption element 1031 and the other end has the sealed chamber 1039 in the same plane. The sealed chamber is also a protruding structure. In this way, the test result reading 1021 is a depressed area on the test device, so that the sealed chamber can be located in the depressed structure to maintain the initial state. Therefore, the depth of depression and the general outline of the testing result reading area 1021 are only required such that the sealed chamber 1039 can be embedded in the depressed test result reading area 1021. Generally, when the sealed chamber 1039 is located in the test result reading area 1021, it is preferred that the chamber is not able to contact the testing area 1082 on the testing element 108 below. In order to avoid this problem. A transparent plate may be arranged at the bottom of the reading area 1021 near the testing area. For example, as shown in FIG. 3, a transparent plate is arranged between two plates 10211, 10212 in the open area at the bottom of the test result reading area. This plate can protect the testing area from damage and also allow reading of the test result. The depressed area 1021 can still serve as the area for accommodating the sealed chamber 1039 containing the treatment liquid. When the depressed area 1021 contains the sealed chamber of the collector, if the collector is removed from the test device, the test result reading area 1021 is exposed, so that the test result can be read.

In some embodiments, an area for receiving the absorption element on the collector is further arranged upstream of the test result reading area 1021. This area is the area used for receiving the absorption element on the collector, and is also provided with an opening on the upper cover. When the upper cover is combined with the bottom plate 101, the bottom plate actually serves as the bottom of the depressed area, the opening serves at an inlet of the absorption element, and the distance between the two plates is the depth of the depressed area. In some embodiments, there are two areas for accommodating the absorption element of the collector. One area is an area where the absorption element stays during the assembly, and in this case, the collector is in the initial first state. The other area is an area where the absorption element stays when the testing is started. As shown in FIG. 3, one area is an area 1023 where the absorption element of the collector stays, docks or is accommodated during the assembly, and when this area accommodates the absorption element of the collector, the sealed chamber 1039 containing the treatment liquid on the collector is located in the test result reading chamber or the depressed area 1021. At this time, the collector covers the upper surface of the upper cover 102. In some embodiments, there is a docking area 10 around the test result reading area 1021 on the upper surface of the upper cover 102. This area allows the handle of the whole collector to cover the upper cover. When the handle covers the upper cover, the absorption element is located in the depression or chamber 1023 (also referred to as the area for accommodating the absorption element). The absorption element may contact the diversion element at the bottom of the chamber or the sample application area 1083 on the testing element 108, and the sealed chamber containing the treatment liquid on the collector is located in the test result reading area 1021. In this case, the collector is accommodated by the accommodating area 10 on the test device. In order to prevent the collector from coming off the upper surface of the test device easily, the upper cover has two snap-fit holes 1028, 1027 distributed on two sides of the test result reading area 1021, and two snap-fit sheets 1037, 1038 are arranged near the tail of the handle on the collector. The snap-fit sheets are bent inward and are elastic. When the handle of the collector covers the test result reading area 1021, the sealed chamber 1039 containing the treatment liquid on the collector 103 is located in the test result reading area 1021, and the snap-fit sheets 1038, 1037 distributed on the two sides of the handle of the collector respectively snap into the snap-fit holes 1028, 1027. Since the snap-fit sheets are elastic inward, they are tightened on the upper cover based on the elastic action. In this case, the collector is located in the initial first state, and at this time, the collector is fixed on the test device, specifically fixed on the surface of the upper cover 102. More specifically, the collector covers the upper side of the test result reading area 1021 and the chamber 1022, and the absorption element is located inside the chamber 1023. The first state of the collector means the state as delivered, or the state in which the collector has not collected the sample, or the state before the testing is started. It can be understood that the first state of the collector on the test device is the state before the testing is started, which is typically the state in which the absorption element of the collector has not absorbed the sample. Of course, the first state of the collector may also be the state in which the collector has left the test device to collect the sample.

In other embodiments, the test device further includes a piercing element display area. Through this area, the piercing element can be seen, and the piercing element can be located in this area without being exposed, which can protect the piercing element. For example, as shown in FIG. 3, FIG. 4, FIG. 5.2 and FIG. 7, the piercing element display area 1024 is located at one end of the test device, the test result reading area 1021 is located at the other end of the test device, and the area for accommodating the absorption element on the collector is located between the two areas 1021, 1024. Actually, the area 1024 containing the piercing element 1011 also has two functions. One function is to contain the piercing element and protect the piercing element from being exposed; and the other function is to allow the sealed chamber containing the treatment liquid on the collector to be located in the area 1024 containing the piercing element, allow the piercing element to pierce the sealed chamber 1039 such that the treatment liquid is released to start testing, and allow the collector containing the chamber 1039 to be accommodated in this area without leaving the test device after the testing is completed. In this specific embodiment, there are two areas 1022, 1023 to accommodate the absorption element. As described above, when the collector is in the first state, the accommodating area 1023 close to the area containing the piercing element accommodates the absorption element 1031. As for the accommodating area 1022 close to the test result reading window, when the collector that has collected a sample starts testing, the absorption element is located in the accommodating area 1022 and contacts the sample application area 1082 of the testing element in this area. Therefore, in some cases, when the collector is in the first state, the absorption element of the collector does not contact the sample application area 1083 on the testing element 108, but contacts the diversion element 1084. When testing is needed, the absorption element of the collector is allowed to contact the sample application area 1083 of the testing element. At this time, the sealed chamber containing the treatment liquid on the collector is allowed to be located in the accommodating chamber 1024. A pressure is applied to the back side of the collector. On the one hand, this pressure allows the film 1040 on the sealed chamber 1039 to be pierced by the piercing elements 1011, 1013 located in the accommodating chamber 1024 such that the treatment liquid is released onto the diversion element 1084 and flows to the sample application area 1083 of the testing element. In this area, the treatment liquid also contacts the absorption element 1031, so that the sample on the absorption element is dissolved or eluted by the treatment liquid. On the other hand, the pressure applied allows the absorption element to be compressed and release the liquid sample onto the sample application area 1083 of the testing element 108. If the absorption element has absorbed or stuck a powder sample, then the treatment liquid flows onto the absorption element 1031 to dissolve the powder sample and then drives the powder sample to flow together to the downstream label area, the testing area and the water absorption area sequentially. In this case, the collector is in the second state in which testing is started. In this state, the absorption element of the collector is located in the accommodating chamber 1022, and the sealed chamber 1039 containing the treatment liquid on the handle is located in the accommodating chamber 1024, such that the test result reading area 1021 is exposed. At this time, the test result can be read through the area 1021. For example, whether lines in the testing area 1087 and the test result control area 1088 are revealed can be read, or the fluorescence intensity on the test line 1087 can be read using a machine. Before testing is started, the collector leaves the accommodating area, and then the absorption element of the collector absorbs or sticks a sample. After the collection of the sample is completed, the collector returns to the accommodating area of the test device, thereby completing the testing. Accordingly, in order to make the whole cover the test device, the area 1024 containing the piercing element is a part of the accommodating area 19. This part includes areas 12, 11 distributed on two sides of the area 1024, a plane 14 close to the area for accommodating the absorption element 1023, and the area 1094 at the tail. These areas are combined to accommodate the handle of the collector. When the handle of the collector is accommodated, the sealed chamber 1039 on the handle is also allowed to be located in the depressed accommodating area 1024. In some embodiments, an area 1099 accommodating the area 1024 is also included. This area can also be covered by the cover plate 104, which can protect the piercing element. In this case, the collector is in the initial state. When the collector is in the second state in which testing is started, the testing area is exposed such that the test result can be read through the area 1021. After the test result is read, the area 1097 can be covered by the cover plate 104, so as to cover the test result. Therefore, the collector has the second state in which testing is started. In this state, the absorption element 1031 of the collector is located in the area 1022, and preferably, contacts the sample application area of the testing element in the area 1022 to start testing. Of course, before the collector is in the second state, it is in a state of collecting a sample, for example, the collector absorbs a liquid sample, or sticks or adsorbs a solid powder sample with the absorption element 1031, or the collector is in the initial first state.

In a specific embodiment of the present invention, when the collector 103 is located in the accommodating chamber 1023 and the test result reading chamber 1021, this accommodating chamber or accommodating area can be called the "first" accommodating area or accommodating chamber. During the testing, the absorption element of the collector is located in the accommodating chamber 1022 and the sealed chamber is located in the accommodating chamber 1024 containing the piercing element, and in this case, the accommodating area formed by the accommodating chamber 1022 and the accommodating chamber 1024 can be called the "second" accommodating area or accommodating chamber. The first and second accommodating areas are different areas on the test device. The "different areas" here refer to different positions on the test device.

It should be particularly noted that the "accommodating area" described in the present invention means that this area can carry, receive, dock and keep the collector on the test device, for example, on the upper 102. These accommodating areas may be in any structure or any form, as long as they can fix the collector to the test device. Particularly, these accommodating areas include the chamber for accommodating the absorption element on the collector and the sealed chamber for accommodating the treatment liquid on the collector. The "accommodating chamber" is one of the specific embodiments of the accommodating area. The chamber typically has a bottom and an opening, and also has a certain depth. These accommodating chambers can allow some structures to be located therein. In the present invention, the absorption element or the sealed chamber containing the treatment liquid on the collector can be allowed to be located in the accommodating chambers, which can reduce the volume and space of the whole device and also play a protection role. For example, the absorption element is located in the accommodating chamber 1023, which prevents the absorption element from being worn or damaged; and the sealed chamber 1039 containing the treatment liquid is located in the test result reading chamber 1021, which prevents the sealed chamber from being destructed or damaged, causing leakage of liquid; and in addition, the test result reading window is covered, which protects the testing area in the chamber 1021 from damage. When the collector is in the initial state, the connecting rod of the handle of the collector and the absorption element also covers the accommodating chamber 1022, which protects part of the sample application area 1083 of the testing element in the chamber 1022 from damage. The "depression area" is also a specific embodiment for accommodating the collector. The whole collector may be accommodated on the test device, or part of the collector may be accommodated and carried by the test device.

It can be understood that in some embodiments, there is only one accommodating chamber between the test result reading chamber 1021 and the chamber 1024 containing the piercing element, and no matter whether the collector is in the first state (contains no sample) or in the second state (contains a sample), the accommodating chamber is used for accommodating the absorption element on the collector. However, the handle of the collector is in different positions, or the sealed chamber on the handle is in different positions when in different states. For example, when the collector is in the first state, the sealed chamber containing the treatment liquid on the handle is located in the test result reading chamber 1021, and the absorption element is located in the accommodating chamber 1022, for example, in the accommodating chamber 1022. However, in this embodiment, there is no accommodating chamber 1023, and the accommodating chamber 1022 contains part of the sample application area 1083 of the testing element 108. In this case, the absorption element on the collector contacts part of the sample application area of the testing element, for example, the absorption element is located on the upper surface of the sample application area. When the accommodating chamber 1023 is absent, the distance between the chamber 1024 containing the piercing element and the accommodating chamber 1022 is equal to the distance between the test result reading chamber 1021 and the accommodating chamber 1022. When testing is needed, the collector is allowed to leave the test device, and a sample is collected by the absorption element. The absorption element containing the sample is allowed to be located in the accommodating chamber 1022 again. At this time, the absorption element containing the sample is in contact with part of the sample application area 1083 on the testing element 108. The sealed chamber containing the treatment liquid is allowed to be located in the chamber 1024 containing the piercing element, so that the piercing element 1011 or 1013 pierces the film 1040 of the sealed chamber. Thereby, the treatment liquid is released to the diversion element 1084. The treatment liquid flows along the diversion element to the downstream sample application pad 1083, contacts the absorption element on the sample application pad, and dissolves or elutes the sample on the absorption element. The treatment liquid drives the sample to flow to the downstream label area and testing area, thereby testing whether the sample contains the analyte. Here, the first accommodating area (the test result reading window or the chamber 1021 and the accommodating chamber 1022) and the second accommodating area (the chamber 1024 containing the piercing element and the accommodating chamber 1022) on the test device share the common accommodating chamber 1022, but the collector is in different directions, which indicates different states and functions of the accommodating chamber. In this case, the first accommodating area and the second accommodating area can be called different positions, i.e., different positions on the test device.

In some embodiments, the present invention provides a novel collector. As shown in FIG. 8 to FIG. 10, one end of the collector carries an absorption element, and the other end carries a chamber containing a treatment liquid. For example, in the specific embodiment shown in FIG. 8 to FIG. 10, the collector 103 has an elongated neck 1035. One end of the neck has an absorption element 1031, and the other end of the neck is connected with a main body of the collector. The width of the main body is greater than the width of the neck. The whole neck is in a sheet structure, or is called a handle of the collector. The absorption element 1031 may be bonded to the collector through glue. However, if the absorption element has a very small volume and only a trace amount of sample needs to be collected, it is difficult for the absorption element to be bonded to the collector through an adhesive agent. Therefore, the present invention provides an elastic clamping element 106. This elastic clip has two opposite faces 1061, 1062 which are connected through elastic elements 1065, 1066 (as shown in FIG. 2 and FIG. 9), and the absorption element is located between the two opposite faces. A plurality of absorption elements, for example, 3 absorption elements as shown in FIG. 9, may be arranged between the two opposite faces, and each absorption element is about 10 mm long and 2 to 3 mm wide. In this way, the three absorption elements are arranged between the opposite faces 1061, 1062, and an absorption head having a length of about 4 to 5 mm is exposed from each absorption element. When the absorption elements arranged in this form are used to absorb a trace amount of sample, the collection of the liquid sample can be ensured. The absorption elements clamped by the elastic elements need to be placed in a chamber, thereby ensuring the fixation of the absorption elements. Therefore, the collector is provided with a chamber 1032 for accommodating the clamping elements 106. The two opposite faces 1061, 1062 are combined under the action of the elastic elements 1065, and then inserted into the chamber 1032, thereby fixing the absorption elements to the collector. The elastic element 1065, which allows the two opposite faces 1061, 1062 to expand in the natural state, overcomes the elastic force so as to allow the two faces to be combined and clamp the absorption elements, and the two faces clamping the absorption elements are inserted into the chamber 1032, so that the absorption element is fixed in the chamber 1032, with one end exposed to take the sample.

When in use, the absorption head is allowed to contact the sample, so that the sample can be collected, absorbed and stuck by the absorption element. When the collector of the present invention is used to collect the sample, the amount of sample collected does not need to be large. For example, if the sample is a liquid, only a few microliters will be enough. The collection head contains the absorption element 1031. The absorption element is fixedly arranged in the chamber 1031 on the absorption head. The absorption element is similar to a toothbrush. A small part of the absorption head is exposed from the chamber, and this part is used for absorbing the liquid sample. When the collector is used for collecting saliva from an oral cavity, the absorption head is allowed to go deep into the oral cavity, and the saliva sample can be adsorbed to the absorption element 1031 by slightly scraping the oral cavity or brushing teeth with the absorption element 1031.

In order to make the clamping element firmly fixed to the collector, the side wall of the chamber is provided with an opening 1034, and one side of the fixing element 106 has a protrusion 1063. When the fixing element is inserted into the chamber 1032, the protrusion 1063 of the fixing element pops out of the opening 1034 on the side wall of the chamber 1032 on the absorption head, so that the fixing element is more fixed in the chamber and the absorption element 1031 is more fixed in the chamber 1032. The parts of the absorption elements exposed out of the chamber are similar to several teeth side by side, which is convenient for scraping saliva off the oral cavity. Thus, in a case of collecting a saliva sample, the exposed part of the absorption element is allowed to slightly contact the oral cavity so as to absorb the saliva sample. In this case, the volume of the saliva sample collected does not need to be too large, and only a few microliters will be enough, for example, 1 to 50 microliters. In the traditional method of collecting saliva, after a sponge head or polyester fiber is put in the mouth, the testee has to keep the sponge head in the mouth a few minutes until the whole collection head is soaked with the saliva, which inevitably makes the testee uncomfortable and repulsed. However, with the collector of the present invention, it is only required to slightly scrape the oral cavity to collect the saliva, which only takes a few seconds. The collector of the present invention only requires a small volume of sample, and only takes a short time of collection, which makes the testee more comfortable. In addition, in actual situations, for example, if the testee is a drug addict or a drug abuse patient who has only a small amount of saliva, the collector of the present invention can be used to complete the drug of abuse testing by collecting only a small amount of sample. Of course, in addition to collecting the liquid sample, the collector of the present invention can also be used for collecting a solid sample, such as a powder sample. By allowing the absorption head to slightly wipe the surface of an appliance, the powder on the surface of the appliance can be stuck to the absorption element. For example, in drug trading places or places where drug addicts live, there is powder on the surfaces of furniture, bedrooms and other appliances, and the absorption element of the present invention may be used to wipe the surfaces so as to take the powder samples.

In some embodiments, an end of the absorption head is further provided with a protruding sheet structure 1042, which is used to be inserted into the fixing structure on the detection chamber so as to fix the collection head to the specific position on the detection chamber. In some embodiments, the detection chamber further includes an accommodating area for accommodating the absorption head, which is used for accommodating the absorption head on the collector. In some embodiments, the collector further includes a plurality of wing areas, such as wing areas 1043, 1045 distributed on two sides of the sealed chamber, and a wing area 1041 at the tail. These wing areas may respectively cover the area 10 of the upper cover of the test device. For example, the wing area 1043 covers the area 1096 of the upper cover, the wing area 1045 covers the area 1095, and the area 1041 at the tail covers the area 18. When the absorption element of the collector is located in the accommodating area 1023, the handle 1035 of the collector covers the accommodating area 1022 and the area 13, thereby covering the upper cover as a whole. In this case, the sealed chamber of the collector is located in the accommodating area 1021.

In some specific embodiments, on the upper cover of the test device, the structure for accommodating the absorption element 1033 is 2 depressed areas in which a beam 10233 is arranged. The beam divides the depressed area into 2 areas. One area is the first receiving area 1022 close to the test result reading area, and the other area is the second receiving area 1024 close to the piercing area. In different cases, these two receiving areas receive the absorption heads or allow the absorption heads to be respectively inserted therein. When the extended sheet structure 1037 of the absorption head is inserted into the receiving area 1022 close to the test result reading window 1021, the extended sheet structure 1042 just abuts against a snap-fit area 1091. At this time, the absorption element that has absorbed the liquid sample needs to start testing, and the sheet structure 1042 is pressed against the snap-fit area 1091. When the collector is pressed down such that the chamber containing the treatment liquid on the collector enters the piercing area, the absorption element can be brought into contact with the sample application area on the test strip, so that the absorption element is compressed. If the absorption element has absorbed the sample, the liquid sample can be released. If there is not enough sample, the absorption element can be brought into close contact with the sample application area. In addition, the sealed chamber containing the treatment liquid on the collector enters the piercing area, and the piercing element 1011 pierces the film 1040 and enters the chamber 1039 containing the treatment liquid, so that the treatment liquid flows onto the diversion element 1084 along the piercing element. Then, the treatment liquid flows through the diversion element 1084 to the downstream sample application area 1083 of the testing element. Part of the treatment liquid enters the absorption element and elutes the liquid on the absorption element, or the treatment liquid is mixed with the sample solution staying on the sample application area, and the mixed solution flows to the downstream label area or testing area, so that the analyte in the sample can be tested and assayed.

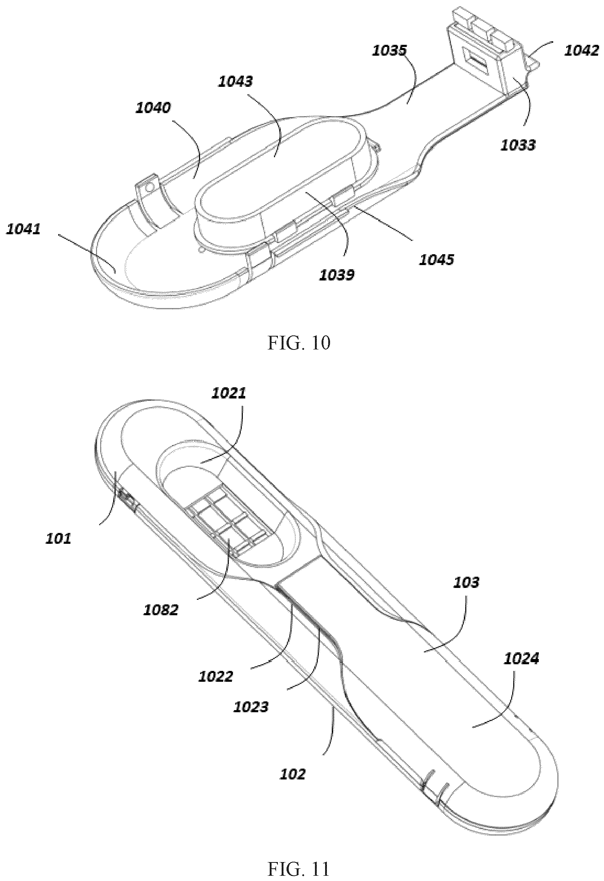

In some embodiments, the main body of the collector includes a chamber 1039, which can be filled with the treatment liquid in advance and then sealed with a sealing film 1043, such as aluminum foil. The sealed chamber 1039 and the absorption element 1031 are in the same plane of the collector, but in different positions, as shown in FIG. 10.

In some embodiments, the test device further includes a covering element 104, and the covering element is provided with buckles 1041, 1042 that are respectively clamped on two sides 10422, 10411 of the bottom plate of the test device. When it is required to remove the covering element, the covering element is pushed out along the arrow, thereby exposing the piercing area 1062. When the collector is used to collect the liquid sample, the collector is moved from the test result reading area to the area containing the pierce structure, so that the absorption element is compressed and the treatment liquid is released, thereby completing the testing. After the testing is completed, the collector is retained on the test device. After the test result is read, the covering element may be used to cover the test result reading area, thereby forming a complete device from the outside. The collector does not need to be discarded separately, but can be discarded together with the test device, thus reducing the inconvenience of separate disposal.

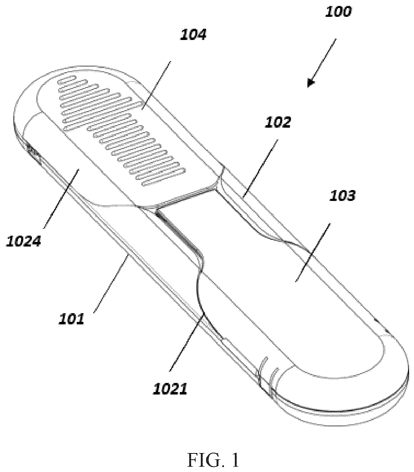

The method of operating or using the test device of the present invention will be described with reference to FIG. 1 and FIG. 11. Firstly, the test device is taken out of the packaging bag. At this time, the covering element 104 covers the area 1024 containing the piercing element, and the collector 103 covers the accommodating area close to the area containing the piercing element. The absorption element of the collector is located in the accommodating chamber 1023, and the sealed chamber containing the treatment liquid on the collector is located in the test result reading area 1021. Thereby, the whole collector covers the upper cover 102 of the test device (as shown in FIG. 1). At this time, the collector is in the initial first state.

When testing is needed, the collector is allowed to leave the test device, the absorption element on the collector is allowed to contact the position where the sample is to be taken, and then scrape, wipe, stick and absorb the sample. Then, the covering element 104 is removed. For example, the covering element 104 is removed by pushing the covering element outward along the direction of the arrow. In addition, after the collector leaves, the test result reading area 1021 is exposed. Then, the collector is allowed to return to the test device. At this time, the absorption element of the collector is allowed to enter the accommodating area 1022, which is a chamber structure. The sample application area 1083 of the testing element is arranged in this area, and the absorption element 1031 on the collector contacts the surface of the sample application area 1083 of the testing element 108. Then, the handle with the sealed chamber is allowed to cover the piercing area 1024. At this time, the sealed chamber is located in the chamber containing the piercing element. The collector is pressed down. At this time, the piercing element 1011 pierces the film 1043 of the sealed chamber and enters the sealed chamber, such that the treatment liquid in the sealed chamber flows to the diversion element 1084. The treatment liquid flows to the sample application area 1083 along the diversion element 1084 and contacts the absorption element, so that the treatment liquid dissolves the sample on the absorption element, and also drives the liquid sample (released to the sample application pad by squeezing the treatment liquid) (if the sample absorbed by the absorption element is the liquid sample) to flow downstream together through the testing area 1082 and finally to the absorption region 1081. The test result is read through the test result reading area 1021 (as shown in FIG. 11). After the testing is completed, the covering element 104 is pushed in from the front to cover the test result reading area 1097, so as to form a complete structure again, which is convenient to discard and dispose.

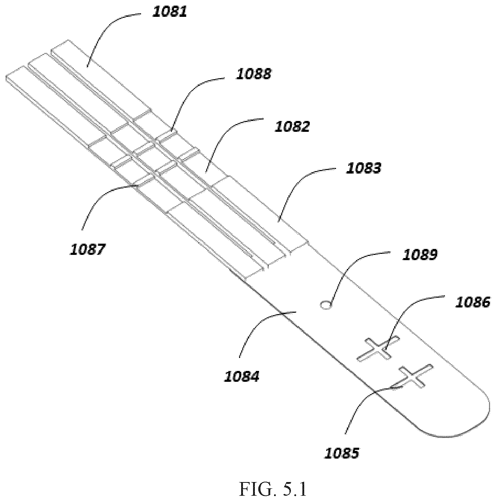

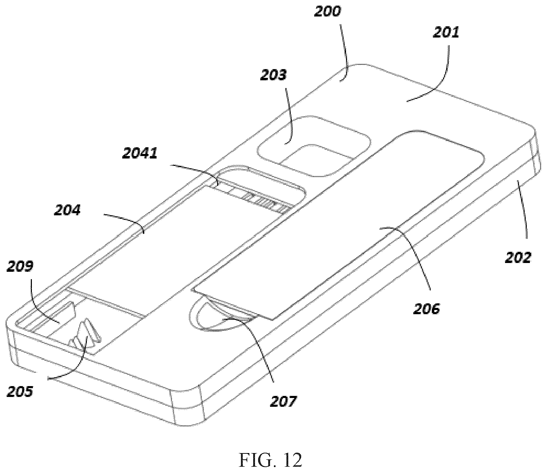

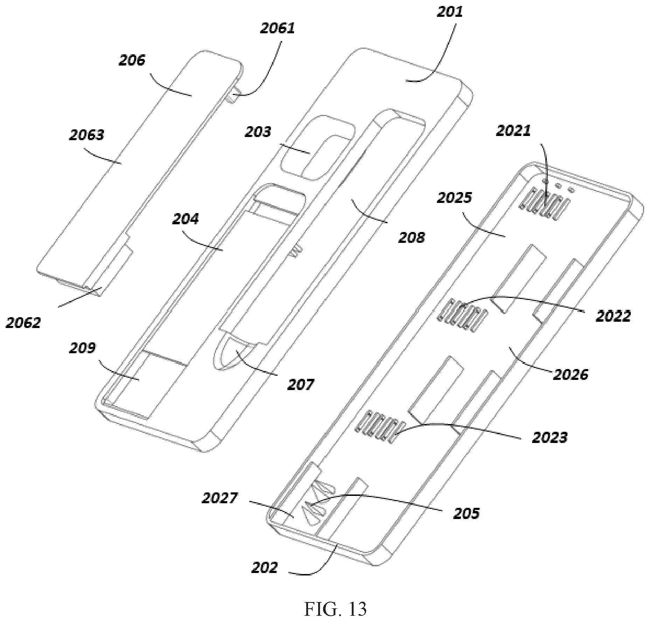

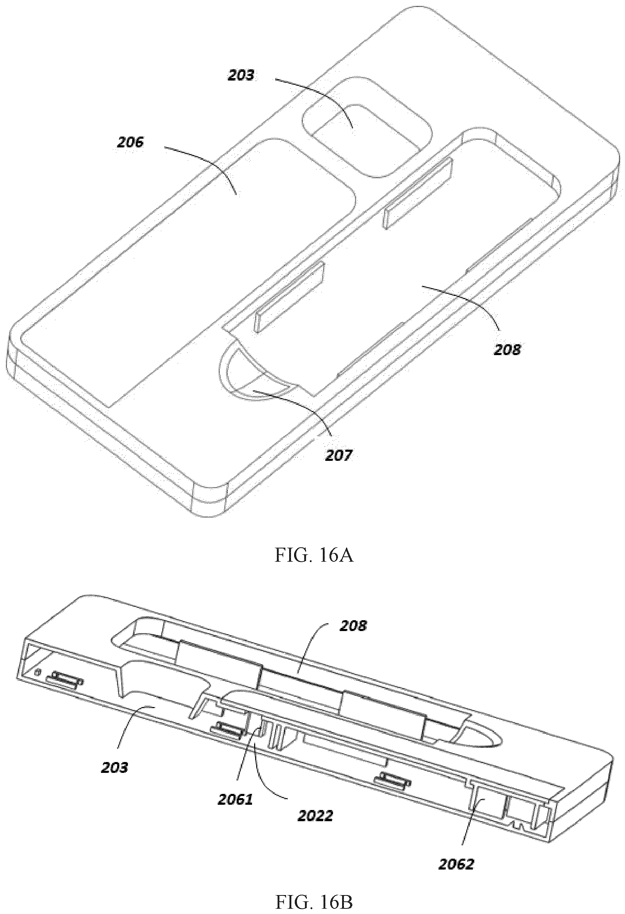

In other embodiments, the collector in the initial state is fixed on the test device, however, after the testing is started, the absolute position of the collector on the test device is changed. For example, as shown in FIG. 12 to FIG. 16, the device includes an upper card 201 and a lower card 202 which are combined to form a complete device 200. The device has an area 208 for specially accommodating the collector, and an area 209 for specially accommodating a test strip, which may also be referred to as a testing area 209. On this testing area, the test strip is located between the upper card and the lower card. The testing area has a test result reading window 203, which shows the testing area of the testing element. The testing element is arranged on the lower card 202, which is divided into an area 2025 for containing the testing element and an area 2026 for containing the collector. The area 2025 for containing the testing element includes a structure area 2021 for containing a water absorption area of the testing element, and a structure area 2022 for containing a sample application area, between which the label area and the testing area of the testing element are arranged. The lower card is also provided with a structure area 2023 for containing a diversion element, and a piercing area 2027 arranged upstream of the area containing the diversion element. Piercing elements 205, 2024 are arranged in the piercing area. A partition 204 is arranged between the piercing area 2027 and the sample application area 2022. The partition serves as a cover between the piercing area and the sample application area, so that two areas are formed on the test device. One is an area 2041 that allows the absorption element of the collector to be inserted, and the other is an area 209 that allows the sealed chamber on the collector to enter. The area 209 includes the piercing elements 205, 2024. FIG. 12 shows an assembled test device. The collector 206 is located in the accommodating area 208 of the collector, and a depressed area 207 is arranged at the tail end of the accommodating area. Before the collector is allowed to leave the test device, a finger can lift one end of the collector through this depressed area, so that the collector can be held by the hand and removed from the test device. In this embodiment, the collector includes several absorption elements 2061, 2064, 2064 which are arranged at one end of the collector 206. Of course, several protruding columns may be arranged on the collector, and absorption elements capable of adsorption or absorption, such as filter paper or sponge, may be bonded to the ends of the columns. The other end of the collector includes a chamber 2062, which contains a plurality of grids 2063, 2066, 2067 communicating with each other. The treatment liquid is injected into the grids in advance, and then the chamber is sealed with a sealing film to form the sealed chamber containing the treatment liquid. During the operation, the collector 206 is allowed to leave the accommodating area 208 to collect the sample, for example, absorb, scrape or wipe the sample with the absorption element. Then, the collector is placed on the testing area 209. The absorption element of the collector is allowed to enter the chamber 2041 and contact the sample application area of the testing element. At the same time, the sealed chamber 2062 containing the treatment liquid at the tail of the collector is allowed to be located in the area 209 containing the piercing element. A pressure is applied to the collector, so that the sealed chamber is pierced by the piercing element to release the treatment liquid. The treatment liquid flows to the diversion element of the testing area. The diversion element guides the treatment liquid to flow to the downstream sample application area. At the same time, the treatment liquid contacts the absorption element 2061 and dissolves the sample therein, and the mixed solution flows to the downstream label pad and then to the testing area. At this time, the test result on the testing area is read through the window 203 (as shown in FIG. 15). In this embodiment of the present invention, the collector has a special accommodating area on the test device. This area does not contain the testing element, the test result reading window and the piercing element, while the testing area contains the testing element, the test result reading window and the piercing element. This arrangement is different from the device and structure shown in FIG. 1 to FIG. 11.

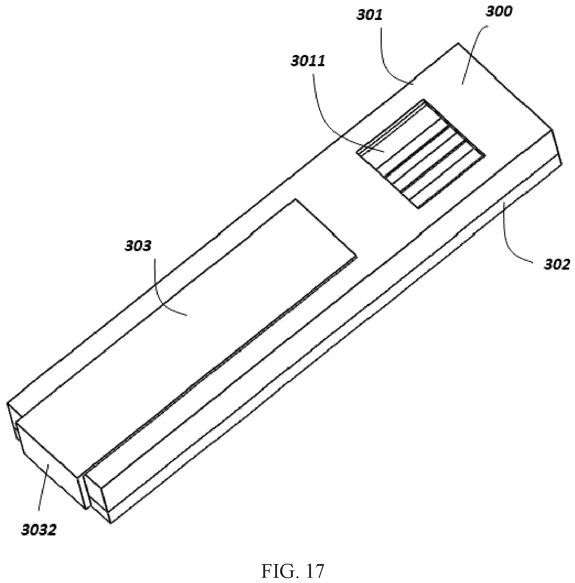

In other specific embodiments, the test device has an area for accommodating the collector. This area is the area in which the collector is in the initial state, and also the area in which testing is started. The accommodation of the collector and the start of the testing are completed in the same area. It can be understood that on the test device, the collector is always accommodated in the same area, but based on the requirements of the testing process, the collector leaves the accommodating area on the test device to collect the sample, and then returns to this area to start testing when testing is needed. In one embodiment, the initial first state in which the collector stays on the test device and the second state in which testing is started after the collection of the sample are completed in the same area, and the positions of the collector in the first state and the second state do not change substantially. For example, in the device shown in FIG. 17 to FIG. 23, the collector is located on the test device. The test device includes an upper card 301 and a lower card 302, between which a testing element is arranged. The test device also includes a test result reading window 3011, below which is the testing area of the testing element. This testing area is the area that shows the test result. The test device further includes a chamber 3012 for accommodating an absorption element, in which a water-absorbent liquid separation element 3022 is arranged. The liquid separation element is in direct contact with the absorption element 3031 on the collector. The liquid separation element 3022 is in liquid communication with a plurality of testing elements. For example, a plurality of testing elements 3031 are arranged under the liquid separation element, and each testing element is connected and in fluid communication with the liquid separation element 3022. It can be understood that the liquid separation element 3022 is a liquid diversion element shared by the plurality of testing elements, and the liquid can respectively flow to the plurality of testing elements on the liquid separation element 3022. In some embodiments, a first diversion element 3023 is further provided upstream of the liquid separation element 3022. The first diversion element 3023 is arranged in an inclined position relative to the liquid separation element 3022, that is, the liquid separation element 3022 is in a higher position, and the diversion element 3023 is in a lower position. In some embodiments, a second diversion element 3024 is arranged upstream of the diversion element 3023. The second diversion element is located below a sealed chamber 304. A chamber 305 is provided, in which a sealed piston chamber 3042 is provided. A treatment liquid is sealed in the piston chamber by a piston. The piston chamber is in fluid communication with the diversion element 3024. Particularly, the diversion element 3024 is located below an opening 3041 at one end of the chamber. The sealed chamber 304 has the opening 3041 at one end and an opening 3043 at the other end, between which a piston 305 is arranged. The piston has a piston rod 3053, and piston gaskets 3052, 3051 are arranged at two ends of the piston rod. The piston gaskets and an inner wall of the chamber 304 form a liquid seal. The treatment liquid is contained in the sealed chamber 3042. The diameter of the piston rod is less than the inner diameter of the chamber 304. Thereby, the sealed chamber 3042 is formed by means of the piston, and the treatment liquid is injected into the sealed chamber in advance. At this time, the piston is in the first position, and the liquid cannot leak out of the sealed chamber 3042. When the piston is pushed, for example, forward to the second position, the piston gasket 3052 moves in the chamber 304 and exposes the opening 3041. The piston gasket 3052 is pushed and located at an end 3048 of the chamber 304. Thus, the treatment liquid in the sealed chamber 3042 flows to the diversion element 3024, and then flows to the liquid separation element 3022 under the capillary action of the diversion element. On this element, the treatment liquid may dissolve and treat the sample on the liquid separation element 3022, or the treatment liquid may contact the absorption element of the collector, and dissolve and treat the sample on the absorption element, so that a mixed solution is formed on the liquid separation element. The mixed solution flows to the downstream testing element, thereby completing the testing or assaying.

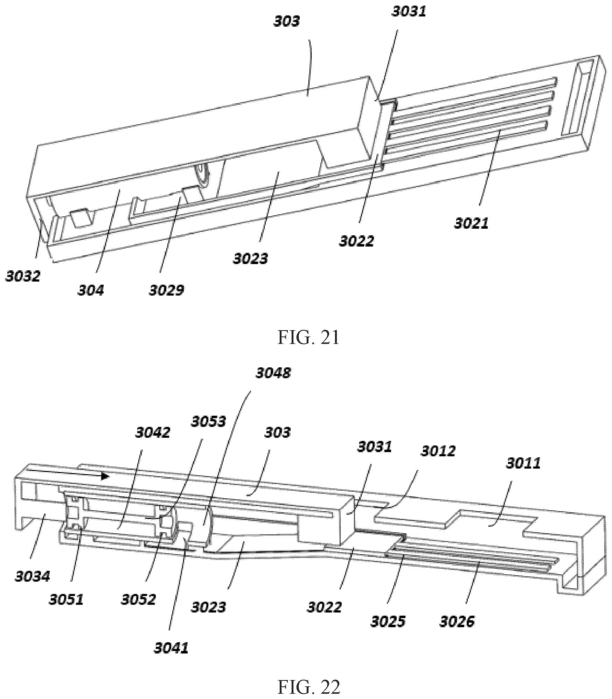

The chamber 304 is relatively fixed on the test device. For example, a plurality of notches 3024, 3025 are provided in the lower card, the chamber 304 is located on the notches, and the upper cover is also provided with a notch 3013. The chamber 304 is clamped between the upper card 301 and the lower card 302 and kept fixed in position. Thus, when the piston 305 moves in the chamber 304, the chamber can be kept fixed. In some embodiments, the piston 305 can be moved in the chamber 304 with the start of the testing by moving the collector. For example, the piston can be moved by moving the collector or a partial structure on the collector by an external force. As shown in FIG. 18, the collector includes a compressible absorption element 3031 at one end, which can be used for collecting a sample, such as a liquid sample or a solid powder sample. The absorption element 3031 may be fixedly connected with one end of a handle in any manner. The other end of the handle 3033 of the collector is provided with a push rod 3034, which is in contact with the piston gasket 3051 of the piston 305 and can push the piston 305 to move in the chamber 304. The push rod 3034 is fixedly connected with a push rod piece 3032 of the handle of the collector. The length of the chamber 3012 for accommodating the 3031 is greater than the width of the absorption element. After the collector has absorbed the sample, the collector returns to the initial position. At this time, the absorption element is in contact with the liquid separation element 3022, but the absorption element can still move forward on the surface of the liquid separation element 3022, for example, there is still a distance 3012 in front of the absorption element for the absorption element to move forward. At this time, the push rod 3034 at the tail end of the handle of the collector is in contact with the piston 305 in the first position (as shown in FIG. 21). An external force is applied to the push rod piece 3032 such that the push rod piece 3032 pushes the push rod to move forward. Here, the movement of the push rod drives the piston to move from the first position to the second position, so that the opening 3041 of the chamber is not sealed, and the treatment liquid flows out of the chamber 304 and then flows through the diversion element 3023 to the liquid separation element 3022. While the push rod moves, the collector also moves forward. At this time, the absorption element also moves on the surface of the liquid separation element 3022 in the accommodating chamber 3012. The movement actually brings the absorption element into contact with the surface of the liquid separation element 3022. If the treatment liquid flows to the liquid separation element 3022, it may contact the absorption element 3031 to elute and dissolve the sample on the absorption element 3031. In addition, in some cases, if the absorption element has absorbed a liquid sample, when the absorption element 3031 comes into contact with the liquid separation element 3022, the absorption element 3031 becomes soft due to the presence of the liquid, and the liquid separation element is still rigid. In this case, when the absorption element 3031 is pushed, it is actually be compressed, so as to release the liquid sample. If the absorption element 3031 has adsorbed a powder sample, the absorption element 3032 rubs the surface of the liquid separation element 3022, so that the powder sample adheres to the liquid separation element. Thus, when the upstream treatment liquid flows to the liquid separation element 3022, the treatment liquid can dissolve the powder sample to form a liquid mixture. Thereby, the liquid mixture flows to the downstream test strip, thereby completing the testing of the analyte (as shown in FIG. 22).

The following specific embodiments are also a part of the present invention.
1. A test device, comprising a detection chamber, wherein the detection chamber is configured to receive a testing element; the testing element is configured to test an analyte in a sample; and the device further comprises a piercing element, the piercing element being in fluid communication with the testing element.
2. The test device according to clause 1, wherein the device comprises a first area and a second area, wherein the first area comprises an area for reading a test result, and the second area comprises an area for accommodating the piercing element.
3. The test device according to clause 3, wherein when testing is not started, the first area is used for receiving a sealed chamber containing a treatment liquid; and when the testing is started, the second area is used for receiving the sealed chamber containing the treatment liquid such that the sealed chamber is pierced by the piercing element to release the treatment liquid.
4. The test device according to clause 2, wherein when testing is not started, the window area for reading a test result is used for receiving a sealed chamber containing a treatment liquid; and when the testing is started, the area containing the piercing element is used for receiving the sealed chamber containing the treatment liquid such that the sealed chamber is pierced by the piercing element to release the treatment liquid.
5. The test device according to clause 4, wherein the device further comprises a collector, wherein the collector comprises an absorption element for collecting a sample, and the sealed chamber containing the treatment liquid is located on the collector.
6. The test device according to clause 5, wherein the test device further comprises an area for accommodating the absorption element on the collector, and the area comprises a sample application area on the testing element.
7. The test device according to clause 6, wherein the area for accommodating the absorption element is located between the first area and the second area.
8. The test device according to clause 7, wherein when the collector is in an initial first state, the window area for reading a test result comprises the sealed chamber containing the treatment liquid; and when the collector is in a second state, the area containing the piercing element comprises the sealed chamber containing the treatment liquid, and the absorption element on the collector is located in the area for accommodating the absorption element.
9. The test device according to clause 8, wherein the area for accommodating the absorption element comprises a first absorption element accommodating area and a second absorption element accommodating area; when the collector is in the first state, the absorption element is located in the second absorption element accommodating area; and when the collector is in the second state, the absorption element is located in the first absorption element accommodating area.
10. The test device according to clause 9, wherein the first absorption element accommodating area is close to the area for accommodating the piercing element, and the second absorption element accommodating area is close the window area for reading a test result.
11. The test device according to clause 10, wherein the first absorption element accommodating area comprises the sample application area of the testing element, and the window area for reading a test result comprises a testing area of the testing element.
12. The test device according to clause 11, wherein the area for accommodating the piercing element is located upstream of the first absorption element accommodating area, the first absorption element accommodating area is located upstream of the second absorption element accommodating area, and the second absorption element accommodating area is located upstream of the testing area on the testing element.
13. The test device according to clause 11, wherein when the collector is in the second state, a sample is collected on the absorption element.
14. The test device according to clause 12, wherein the area containing the piercing element is in fluid communication with the sample application area on the testing element through a diversion element.
15. The test device according to clause 13, wherein the sample is a liquid sample or a solid powder sample.
16. The test device according to clause 15, wherein the liquid is a saliva or urine sample; and the powder sample is a drug of abuse powder sample.
17. The test device according to clause 16, wherein the testing element is a lateral flow testing element.
18. A test device, comprising a detection chamber, wherein the detection chamber is used for receiving a testing element; the testing element is configured to test an analyte in a sample; and the device further comprises a collector, and the collector comprises an absorption element for collecting the sample, wherein the collector has a first state and a second state on the test device.
19. The device according to clause 18, wherein when the collector is in the first state, the collector is in a state prior to collecting a sample; and when the collector is in the second state, the collector is in a state in which a liquid sample is collected and testing is started.
20. The device according to clause 19, wherein the device comprises a first area and a second area for accommodating the collector; when the collector is in the first state, the collector is located in the first area on the test device; and when the collector is in the second state, the collector is located in the second area on the test device.
21. The device according to clause 20, wherein the first area comprises a window area for reading a test result, and the second area comprises an area containing a piercing element.
22. The device according to clause 21, wherein the collector comprises a sealed chamber containing a treatment liquid; when the collector is in the first state, the sealed chamber is located in the window area for reading a test result; and when the collector is in the second state, the sealed chamber is located in the area containing the piercing element.
23. The device according to clause 22, wherein the test device further comprises an area for accommodating the absorption element on the collector, and the area for accommodating the absorption element is located between the window area for reading a test result and the area containing the piercing element.
24. A test device, comprising a detection chamber, wherein the detection chamber is used for receiving a testing element; the testing element is configured to test an analyte in a sample; and the device further comprises a collector, and the collector comprises an absorption element for collecting the sample, wherein the test device comprises a first area for accommodating the collector such that the collector is detachably combined with the test device.
25. The device according to clause 24, wherein the collector has a first state and a second state, wherein in the first state, the collector does not leave the test device, and the absorption element does not contain the sample; and in the second state, the collector is located on the test device, and the absorption element contains the sample.
26. The device according to clause 25, wherein when the collector is in the first state or the second state, the collector is located in the first area.
27. The device according to clause 26, wherein the first area comprises an area for accommodating the absorption element of the collector; and when the collector is in the first state or the second state, the absorption element is located in the area for accommodating the absorption element of the collector, or when the collector is in the first state or the second state, the absorption element is located in the same area for accommodating the absorption element of the collector.
28. The device according to clause 26, wherein the area for accommodating the absorption element of the collector comprises a sample application area of the testing element, or a liquid separation element in fluid communication with the testing element.
29. The device according to clause 28, wherein the absorption element is in contact with the sample application area in the area for accommodating the absorption element or in contact with the liquid separation element.
30. The device according to clause 29, wherein when the collector is in the second state, the collector has a first position and a second position on the test device.
31. The device according to clause 30, wherein the device further comprises a sealed chamber containing a treatment liquid, the sealed chamber containing a movable piston, and the piston having a first position and a second position in the sealed chamber.
32. The device according to clause 31, wherein when the collector is in the first position, the collector contacts the piston, and at this time, the piston is in the first position; and when the collector is in the second position, the collector contacts the piston, and the piston is in the second position.
33. The device according to clause 31, wherein when the piston is in the first position, the treatment liquid is in a sealed state, and when the piston is in the second position, the treatment liquid is flowable out of the chamber.
34. The device according to clause 33, wherein the sealed chamber containing the treatment liquid is located in the sample application area or upstream of the liquid separation element.
35. The device according to clause 34, wherein the test device further comprises a diversion element, the diversion element making the sealed chamber in contact with the sample application area or in fluid communication with the liquid separation element.
36. A collector, comprising a collection head provided with an absorption element, wherein the collector is further provided with a sealed chamber containing a treatment liquid.
37. The collector according to clause 36, wherein the absorption element of the collector is fixed in the chamber of the collector, and a part of the absorption element is exposed to absorb a liquid sample.
38. The collector according to clause 37, wherein the absorption element is fixed by a fixing element, and the fixing element is inserted into the chamber such that the absorption element is fixed in the chamber.
39. The collector according to clause 38, wherein the fixing element is elastic, and the absorption element is fixed into the chamber through an elastic fixing structure.
40. The collector according to clause 39, wherein the absorption element is capable of absorbing a trace amount of sample, such as saliva, slobber or other liquid samples, from an oral cavity.
41. The collector according to clause 40, wherein the collector has two faces, and the absorption element and the sealed chamber containing the treatment liquid are located on the same face.
42. The collector according to clause 40, wherein the trace amount comprises 1 to 100 microliters.
43. The collector according to clause 40, wherein the sample comprises a solid powder sample.
44. A method for testing an analyte in a sample, comprising:
   providing a device, comprising: a detection chamber, the detection chamber being used for receiving a testing element, and the testing element being configured to test an analyte in a liquid sample; and a sample collector, comprising a sample absorption element and a sealed chamber containing a treatment liquid, the collector being accommodated by first and second accommodating areas located on the test device such that the collector is detachably combined with the test device;
   wherein when the collector is located in the first accommodating area, the absorption element of the collector does not contain the sample.
45. The method according to clause 44, wherein when the collector is located in the second accommodating area, the absorption element contains the sample.
46. The method according to clause 45, wherein the first accommodating area is allowed to comprise a window area for reading a test result, and the sealed chamber is allowed to be located in the window area for reading a test result; and the second accommodating area is allowed to comprise an area containing a piercing element, the sealed chamber is allowed to be located in the area containing the piercing element, and the piercing element is allowed to pierce the sealed chamber such that the treatment liquid is released.
47. The method according to clause 45, wherein the window area and the area containing the piercing element further comprise first and second absorption element accommodating areas; when the sealed chamber is located in the window area for reading a test result, the absorption element is located in the second absorption element accommodating area; and when the sealed chamber is located in the area containing the piercing element, the absorption element is located in the first absorption element accommodating area.
48. The method according to clause 47, wherein the area containing the piercing element is allowed to be located upstream of the second absorption element accommodating area, the second absorption element accommodating area is allowed to be located upstream of the first absorption element accommodating area, and the first absorption element accommodating area is allowed to be located upstream of the window area for reading a test result.
49. The method according to clause 48, wherein the first absorption element accommodating area is allowed to comprise a sample application area of the testing element such that the absorption element contacts the sample application area.
50. A method for testing an analyte in a sample, comprising:
   providing a device, comprising: a detection chamber, the detection chamber being used for receiving a testing element, and the testing element being configured to test an analyte in a liquid sample; and a sample collector, comprising a sample absorption element and a sealed chamber containing a treatment liquid, the collector being accommodated by first and second accommodating areas located on the test device such that the collector is detachably combined with the test device;
   wherein the collector is allowed to have a first state and a second state, wherein when the collector is in the first state, the collector is located in the first accommodating area, and when the collector is in the second state, the collector is located in the second accommodating area.
51. The method according to clause 50, wherein when the collector is located in the first accommodating area, the absorption element of the collector does not contain the sample.
52. The method according to clause 51, wherein when the collector is located in the second accommodating area, the absorption element contains the sample and testing is started.
53. The method according to clause 52, wherein the first accommodating area is allowed to comprise a window area for reading a test result, and the second accommodating area is allowed to comprise an area containing a piercing element; and the sealed chamber is allowed to be located in the area containing the piercing element, and the piercing element is allowed to pierce the sealed chamber such that the treatment liquid is released.
54. The method according to clause 53, wherein when the collector is in the first state, the sealed chamber is allowed to be located in the window area for reading a rest result; and when the collector is in the second state, the sealed chamber is allowed to be located in the area containing the piercing element, and the piercing element is allowed to pierce the sealed chamber such that the treatment liquid is released.
55. The method according to clause 54, wherein the window area and the area containing the piercing element further comprise first and second absorption element accommodating areas.
56. The method according to clause 55, wherein when the collector is in the first state, the sealed chamber is allowed to be located in the second absorption element accommodating area; and when the collector is in the second state, the sealed chamber is allowed to be located in the area containing the piercing element, and the piercing element is allowed to pierce the sealed chamber such that the treatment liquid is released.
57. The method according to clause 56, wherein the area containing the piercing element is allowed to be located upstream of the second absorption element accommodating area, the second absorption element accommodating area is allowed to be located upstream of the first absorption element accommodating area, and the first absorption element accommodating area is allowed to be located upstream of the window area for reading a test result.
58. The method according to clause 57, wherein the first absorption element accommodating area is allowed to comprise a sample application area of the testing element such that the absorption element contacts the sample application area.
59. The method according to clause 58, wherein the collector is allowed to be in the first state, the collector is allowed to leave the test device, and the absorption element is allowed to collect a sample such as a liquid sample or a solid powder sample.
60. The method according to clause 59, wherein the collector is allowed to be in the second state, and the collector is allowed to be located in the second accommodating area on the test device, such that the released treatment liquid flows to the downstream first absorption element accommodating area to contact the absorption element and is mixed with the sample to form a mixed solution, and the mixed solution flows to the downstream testing area.
61. The method according to clause 60, wherein a test result is read through the window area for reading a test result.

All the patents and publications mentioned in the description of the present invention indicate that these are public technologies in the art and can be used by the present invention. All the patents and publications cited herein are listed in the references, just as each publication is specifically referenced separately. The present invention described herein can be realized in the absence of any one element or multiple elements, one restriction or multiple restrictions, where the limitation is not specifically described here. For example, the terms "comprising", "essentially consisting of and "consisting of in each example herein may be replaced by the rest 2 terms. The term "alan" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the present invention and appended claims. It can be understood that the embodiments described in the present invention are some preferred embodiments and features. Any person of ordinary skill in the art can make some modifications and changes according to the spirit of the description of the present invention. These modifications and changes are also considered to fall within the scope of the present invention and the scope limited by independent claims and dependent claims.

## Claims

1. A test device, comprising a detection chamber, wherein the detection chamber is configured to receive a testing element; the testing element is configured to test an analyte in a sample; and the device further comprises a piercing element, the piercing element being in fluid communication with the testing element.

2. The test device according to claim 1, wherein the device comprises a first area and a second area, wherein the first area comprises an area for reading a test result, and the second area comprises an area for accommodating the piercing element.

3. The test device according to one of claims 1-2, wherein when testing is not started, the first area is used for receiving a sealed chamber containing a treatment liquid; and when the testing is started, the second area is used for receiving the sealed chamber containing the treatment liquid such that the sealed chamber is pierced by the piercing element to release the treatment liquid.

4. The test device according to claim 2, wherein when testing is not started, the window area for reading a test result is used for receiving a sealed chamber containing a treatment liquid; and when the testing is started, the area containing the piercing element is used for receiving the sealed chamber containing the treatment liquid such that the sealed chamber is pierced by the piercing element to release the treatment liquid.

5. The test device according to one of claims 1-4, wherein the device further comprises a collector, wherein the collector comprises an absorption element for collecting a sample, and the sealed chamber containing the treatment liquid is located on the collector.

6. The test device according to one of claims 1-5, wherein the test device further comprises an area for accommodating the absorption element on the collector, and the area comprises a sample application area on the testing element.

7. The test device according to claim 6, wherein the area for accommodating the absorption element is located between the first area and the second area.

8. The test device according to one of claims 1-7, wherein when the collector is in an initial first state, the window area for reading a test result comprises the sealed chamber containing the treatment liquid; and when the collector is in a second state, the area containing the piercing element comprises the sealed chamber containing the treatment liquid, and the absorption element on the collector is located in the area for accommodating the absorption element.

9. The test device according to one of claims 6-8, wherein the area for accommodating the absorption element comprises a first absorption element accommodating area and a second absorption element accommodating area; when the collector is in the first state, the absorption element is located in the second absorption element accommodating area; and when the collector is in the second state, the absorption element is located in the first absorption element accommodating area.

10. The test device according to claim 9, wherein the first absorption element accommodating area is close to the area for accommodating the piercing element, and the second absorption element accommodating area is close the window area for reading a test result.

11. The test device according to claim 10, wherein the first absorption element accommodating area comprises the sample application area of the testing element, and the window area for reading a test result comprises a testing area of the testing element.

12. The test device according to claim 11, wherein the area for accommodating the piercing element is located upstream of the first absorption element accommodating area, the first absorption element accommodating area is located upstream of the second absorption element accommodating area, and the second absorption element accommodating area is located upstream of the testing area on the testing element.

13. The test device according to claim 12, wherein the sample is a liquid sample or a solid powder sample.

14. A test device, comprising a detection chamber, wherein the detection chamber is used for receiving a testing element; the testing element is configured to test an analyte in a sample; and the device further comprises a collector, and the collector comprises an absorption element for collecting the sample, wherein the collector has a first state and a second state on the test device.

15. The device according to claim 14, wherein when the collector is in the first state, the collector is in a state prior to collecting a sample; and when the collector is in the second state, the collector is in a state in which a liquid sample is collected and testing is started.

16. The device according to one of claims 14-15, wherein the device comprises a first area and a second area for accommodating the collector; when the collector is in the first state, the collector is located in the first area on the test device; and when the collector is in the second state, the collector is located in the second area on the test device.

17. The device according to claim 16, wherein the first area comprises a window area for reading a test result, and the second area comprises an area containing a piercing element.

18. The device according to claim 17, wherein the collector comprises a sealed chamber containing a treatment liquid; when the collector is in the first state, the sealed chamber is located in the window area for reading a test result; and when the collector is in the second state, the sealed chamber is located in the area containing the piercing element.

19. The device according to one of claims 14-18, wherein the test device further comprises an area for accommodating the absorption element on the collector, and the area for accommodating the absorption element is located between the window area for reading a test result and the area containing the piercing element.
